Europäisches Patentamt
European Patent Office
Office européen des brevets

(19) (11) Publication number: **0 248 984 A2**

# (12) EUROPEAN PATENT APPLICATION

(21) Application number: 87103955.8

(22) Date of filing: 18.03.87

(51) Int.Cl.³: C 12 N 15/00
C 12 N 1/20
//C12P17/18, C12P19/28

(30) Priority: 19.03.86 US 841464
11.04.86 US 850963

(43) Date of publication of application:
16.12.87 Bulletin 87/51

(84) Designated Contracting States:
AT BE CH DE ES FR GB GR IT LI LU NL SE

(71) Applicant: THE STATE OF OREGON ACTING BY AND THROUGH THE OREGON STATEBOARD OF HIGHER EDUCATION ON BEHALF OF OREGON STATE UNIVERSITY
P.O. Box 3175
Eugene Oregon 97403(US)

(72) Inventor: Morris, Roy O.
7620 Hoodview Circle
Corvallis, Oregon 97330(US)

(72) Inventor: Regier, Dean A.
727-1/2 No. 100th Street
Seattle, Washington 96133(US)

(74) Representative: Brown, John David et al,
FORRESTER & BOEHMERT Widenmayerstrasse 4/I
D-8000 München 22(DE)

## (54) Production of cytokinins by microorganisms.

(57) Cytokinins, particularly trans-zeatin, are produced by bacterial cells containing plasmids, produced by recombinant DNA techniques, with insert DNA (*tmr* and/or *tzs*) isolated from Ti plasmids of *Agrobacterium tumefaciens* or with insert DNA isolated from a plasmid of *Pseudomonas syringae* pv. *savastanoi*. Three such recombinant plasmids, pCK101, pTZ120 and pIP192, are obtainable from ATCC 53438, 53437, and 53436, respectively, and are produced by combining a cloning vector with *tmr* and *tzs* (pCK101), the *tzs* (pTZ120), and with *tmr* (pIP192). When both *tmr* and *tzs* are included in the same plasmid, there is a substantial elevation in trans-zeatin yield. Another recombinant is pPS003, obtainable from ATCC 53439, which was made by combining a cloning vector with *ptz* from the *P. savastanoi* plasmid.

EP 0 248 984 A2

# PRODUCTION OF CYTOKININS BY MICROORGANISMS

## BRIEF SUMMARY OF THE INVENTION

This invention relates to the production of plant growth hormones, and more specifically to the production of cytokinins by genetically modified microorganisms.

Cytokinins are naturally occuring phytohormones characterized primarily by their ability to induce cell division in culture. They are derivatives of adenine or adenosine in which the exocyclic amino group at $N^6$ has been modified by attachment of a dimethylallyl side chain, giving the parent compound iso-pentenyladenine (iP) and its riboside (iPA). The dimethylallyl side chain can be hydroxylated to give trans-zeatin (Z) or its riboside, trans-ribosylzeatin (ZR). In addition, the exocyclic double bond can be reduced to give dihydrozeatin (DHZ) or ribosyl dihydrozeatin (DHZR). All six compounds; iP, iPA, Z, ZR, DHZ and DHZR are biologically active and occur naturally.

Cytokinin structures include:

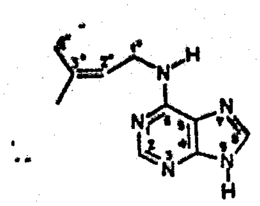

isopentenyladenine iP

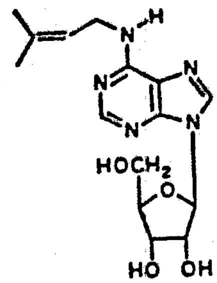

isopentenyladenosine iPA

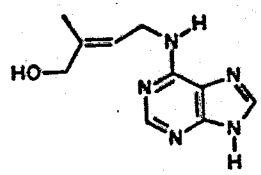

*trans*-zeatin Z

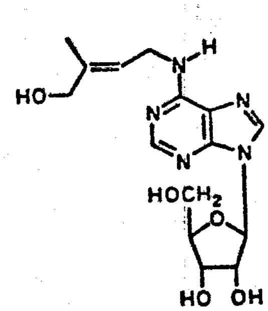

ribosyl-*trans*-zeatin ZR

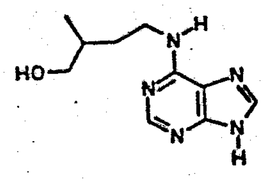

dihydrozeatin DHZ

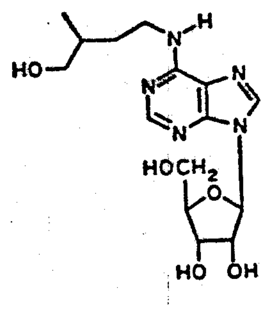

ribosyldihydrozeatin DHZR

A number of diverse developmental events in plants are influenced by cytokinins. These include stimulation of cell division in culture, release of lateral buds from apical dominance, chloroplast differentiation, stimulation of leaf growth and inhibition of leaf senescence as described by D. S. Letham in Phytohormones and Related Compounds - A Comprehensive Treatise, eds. Letham et al. (Elsevier, 1978), p. 205. In addition to these basic physiological functions, application of cytokinins improves post-harvest quality of leafy vegetables (e.g. broccoli, brussels sprouts) as described by R. J. Weaver

in Plant Growth Substances in Agriculture (1972), pp. 296-304. It can induce desirable plant habit in conifers used as Cristmas trees (Abbott Labs, "Proshear" Label, 1985).

Cytokinins have been synthesized chemically by a number of different routes. Zeatin was originally synthesized as described in Shaw et al., J. Chem. Soc., 6:921 (1966). The side chain, 4-hydroxy-3-methyl-but-2-enylamine, was prepared from bromotiglate and then condensed with 6-chloropurine. The procedure is complex, multi-stage and the yields are poor. An alternate synthesis from isoprene described in Leonard et al., J. Am. Chem. Soc., 73:3056-3058 (1971) presents similar problems. A recent synthesis reported in David et al., Tet. Lett., 23:1817-1820 (1982) has improved the yield but is still complex. No chemical synthesis proposed to date has been economically attractive.

In principle, cytokinins can be produced by fermentation. Early studies showed that a number of different organisms produce high levels of cytokinins when grown in culture. For example, the mycorrhizal fungus *Rhizopogon roseolus* was shown, by Miller, Science, 157:1055-1057 (1967) to produce zeatin. Similarly, the plant pathogenic bacteria *Corynebacterium fascians* and *Agrobacterium tumefaciens* secrete cytokinins into the culture medium as described in Scarborough et al., Proc. Natl. Acad. Sci. USA, 70:3825 (1973) and Regier et al., Biochem. Biophys. Res. Commun., 104:1560-1566 (1982). Specifically, *Agrobacterium tumefaciens* strains which harbor nopaline Ti plasmids secrete modest levels of zeatin into the growth medium (Regier et al., 1982).

The biosynthetic pathway for cytokinins is not yet fully understood but probably is as follows (Letham et al. in Plant Growth Substances, ed. Wareing (London, 1983), pp. 143-153).

$$\begin{array}{ccccccc} DMAPP + 5'AMP & \rightarrow & iPMP & \rightarrow & iPA & \rightarrow & iP \\ & & \downarrow & & \downarrow & & \downarrow \\ & & ZMP & \rightarrow & ZR & \rightarrow & Z \end{array} \qquad (1)$$

Dimethylallylpyrophosphate (DMAPP) provides the five-carbon side chain which condenses with 5'AMP to give *iso*-pentenyladenosine phosphate (iPMP). Dephosphorylation and deribosylation then lead sequentially to iPA and iP. Hydroxylation could occur at any point to give zeatin monophosphate (ZMP), zeatin riboside (ZR) or zeatin (Z).

Recently it has been found that genes specifying the enzymes which control this biosynthesis are present in *Agrobacterium tumefaciens*. For example, crown gall tissue, which results from the transformation of plant cells by the integration of T-DNA from *Agrobacterium tumefaciens* Ti plasmids, has elevated levels of cytokinins as described in Akiyoshi et al., Proc. Natl. Acad. Sci. USA, 30:407-416 (1983). Tumors also have elevated levels of the first enzyme in the cytokinin pathway, dimethylallylpyrophosphate 5'AMP transferase as discussed by R. O. Morris et al. in "Cytokinin Metabolism in Relation to Tumor Induction by *Agrobacterium tumefaciens*", Plant Growth Substances, ed. Wareing (London,

1983), pp. 175-183. In a subsequent study of the T-DNA, Barry et al. showed that the tmr locus codes for DMA transferase, as described in Proc. Natl. Acad. Sci. USA, 81:4776-4780 (1984).

A second locus, (tzs), which codes for zeatin production, is present in nopaline Ti plasmids (Regier et al.). It is not, like the tmr locus, inside the T-DNA and is found only in nopaline plasmids. When present in A. tumefaciens it confers on it the ability to secrete zeatin into the culture medium. The nature of the tzs gene function is not clear. It does have DMA transferase activity as does tmr, but may also have a hydroxylation function. That is to say, it may also catalyze the second of the processes noted in Formula (1).

A new cytokinin, ribosyl-1"-methylzeatin, has now been discovered. It is described by Surico et al. in "A New Cytokinin from the Culture Filtrate of Pseudomonas syringae pv. savastanoi" Phytochem. 24:1449-1502 (1985). That new compound is produced, along with other cytokinins, by some Pseudomonas syringae pv. savastanoi (P. savastanoi) strains.

The production of cytokinins, in commercial quantities, would be very useful, but fermentation of Agrobacterium tumefaciens is not pratical because the levels produced are too low (200-400 ng/L of zeatin) to be economically viable.

Likewise, fermentation of savastanoi is not pratical. P. savastanoi is a relatively unstable organism and is difficult to grow in large quantities.

It has now been discovered that high yields of desired cytokinins can be obtained from an easily fermented bacterial species, such as E. coli. This is accomplished by providing, in the bacterial cells, plasmids with certain insert DNA obtained from a Ti plasmid of A. tumefeciens.

It has also been discovered that cytokinins, including *trans*-zeatin, iso-pentenyladenine and their respective $N^9$-ribosyl derivatives, can be obtained from an easily fermented bacterial species, such as *E. coli*. This is accomplished by providing, in the *E. coli* cells, plasmids with certain insert DNA (ptz) obtained from a plasmid of *P. savastanoi*. It will thus be possible to obtain cytokinin yields for in excess of those obtained from naturally occurring organisms.

## BRIEF DESCRIPTION OF THE DRAWINGS

In the drawings:

FIG. 1 is a diagram showing a map of the plasmid pTiC58 and the steps by which desired *tzs* subclones are obtained;

FIG. 2 is a series of graphs which show the relative zeatin production of *E. coli* with and without a plasmid containing the *tzs* gene code;

FIG. 3 is a diagram showing the sequencing strategy of *tzs*;

FIG. 4 is a diagram showing the sequence of *tzs*;

FIG. 5 is a diagram showing the nucleotide sequence homology between the *tmr* and *tzs* gene codes;

FIG. 6 is a diagram showing the amino acid sequence homology of polypeptides coded for by *tmr* and *tzs*;

FIG. 7 is a diagram showing a map of the plasmid pTiAch5 and the steps by which desired *tmr* subclones are obtained;

FIG. 8 is a map of the plasmid pCK101;

FIG. 9 is a diagram showing the mass spectra and structure of permethylated and perdeuteromethylated cytokinins from P. savastanoi strain EW 1006;

FIG. 10 is a diagram showing restriction maps of the plasmid pPS001 and subclones pPS002 and pPS003;

FIG. 11 is a diagram showing restriction maps of the cloned 5.2 kb fragment of pPS003 and the ptz-containing region thereof; and

FIG. 12 is the DNA sequence of the 1500-bp region containing the ptz gene.

## DETAILED DESCRIPTION

To provide an effective method of producing cytokinins (particularly trans-zeatin) by fermentation, a series of experiments was conducted to locate and characterize tzs, to create new plasmids containing the tzs gene code, and to obtain expression of cytokinins from E. coli containing the plasmids. Unexpectedly high expression was obtained when both the tzs and tmr codes were present in a single plasmid. Also, a series of experiments was conducted to locate and characterize a gene code (ptz) for biosynthesis of the cytokinins. New plasmids containing the ptz gene code were created, and cytokinins were expressed from E. coli containing the plasmids.

The following definitions may be useful in understanding the terminology of this disclosure:

Replicon - A genetic element which is capable of independent replication. This may include a plasmid, cosmid, bacteriophage DNA, or chromosome.

tmr - A designation for a gene which codes for a cytokinin biosynthetic enzyme and which can be found in the Eco RI fragment 7 of the Agrobacterium tumefaciens Ti plasmid

tzs - A designation for a gene which codes for a cytokinin biosynthetic enzyme and which can be found in or near the vir region of the Agrobacterium tumefaciens Ti plasmid pTiC58.

ptz - A designation for a gene which codes for a cytokinin biosynthetic enzyme and which can be found in the larger (105 kb) plasmid of P. savastanoi strain 1006.

Substantially homologous - A high degree of homology between the sequences of two or more DNA molecules which can be tested for by determining whether the DNA molecules in question hybridize to each other under stringent conditions, such as those set forth in Bethesda Research Laboratories, DNA Detection System Instruction Manual (Catalogue No. 8239SA), pp. 8-9 (1984). See also Leary et al., Proc. Natl. Acad. Sci. USA, 80:4045-4049 (1983), modifying the procedures of Wahl et al., Proc. Natl. Acad. Sci. USA, 76:3683-3687 (1979).

## I. TZS

### The tzs Gene Codes for Cytokinin Biosynthesis and When Expressed in E. coli Results in the Secretion of Zeatin.

The cloning of tzs from pTiC58 and expression in E. coli was achieved by the following procedure. In order to locate and characterize tzs, a library of fragments of DNA from the A. tumefaciens Ti plasmid pTiC58 was prepared in the cosmid vector pVK102 of Knauf et al., Plasmid, 8:45-54 (1982). pTiC58 was partially digested by the method of Hamilton et al., Experientia, 27:229-230 (1971), with HindIII. After sizing, the fragments were ligated to HindIII-cut phosphatase-treated pVK102.

E. coli HB101 [F⁻, hsd S20($r_{B-}$, $m_B^-$), sup E44, ara 14, gal K2, lac Y1, pro A2, rps L20 (Smr), xyl 5, mtl 1, rec A13, λ⁻, described in Boyer et al., J. Mol. Biol. 41:459-472 (1969)] was packaged and transfected, in vitro, by the procedure of Knauf et al. Hybrid plasmids were characterized by treatment with BamHI, EcoRI, KpnI, or HindIII endonucleases. From this information and the known map of pTiC58 developed by DePicker et al., Plasmid, 3:193-211 (1980), it was determined that the library contained a set of clones representing the entire pTiC58 molecule as shown at A in FIG. 1.

Strains from this library were tested for their ability to secrete trans-zeatin. Each cosmid was transferred conjugatively from E. coli HB101 to A. tumefaciens A136, a C58 derivative lacking the entire Ti plasmid, as shown in Watson et al., J. Bacteriol., 123:255-264 (1975), and the culture fluids from the transconjugants were analyzed directly by radioimmunoassay for the presence of trans-zeatin using the method of MacDonald et al., J. Chromatog., 214:101-109 (1982). All strains which secreted trans-zeatin contained cloned fragments from within or near the vir region of pTiC58, discussed in Engler et al., J. Mol. Biol., 152:183-208 (1981). A comparison of zeatin prodution (or the lack of it) by individual clones indicated that tzs is located within HindIII fragment 9, as shown in FIG. 1.

The tzs gene was expressed not only in Agrobacterium tumefaciens but in E. coli as well. Radioimmunoassay of culture fluids of the E. coli library clones bearing the tzs gene indicated that they produced trans-zeatin. One such clone, HB101 (pDR28), was the source of DNA for the subclone constructions shown at B in FIG. 1. Complete digestion of pDR28 with HindIII, followed by treatment with T4 DNA ligase,

gave derivative clones containing single HindIII fragments from pDR28. Transformation of HB101 by this DNA and subsequent analysis showed that only those clones bearing HindIII fragment 9 secreted trans-zeatin, confirming that tzs is located in that fragment.

As shown in FIG. 1, HindIII fragment 9 was purified from one of these subclones, pTZ110, and treated with HpaI. Trans-zeatin-secreting subclones were recovered by ligating the HindIII fragment 9 to pUC8, previously cut with HindIII and HincII by the method of Viera et al., Gene, 19:259-268 (1982), and transforming the resulting plasmid using E. coli JM103. The inserted DNA from one of these tzs-containing plasmids, pTZ120, was a 1.4 kb HpaI-HindIII fragment which is right-most in HindIII fragment 9, as shown at B in FIG. 1. Construction of pTZ121, a plasmid containing tzs in the opposite orientation with respect to the lac promoter was accomplished by excising the tzs fragment from pTZ120 with HindIII and BamHI and ligating it into pUC9. Trans-zeatin was detected in the culture fluids of HB101 bearing either pTZ120 or pTZ121.

Measurements of cytokinins secreted by $tzs^+$ E. coli HB101 was accomplished as follows. Cytokinins present in culture media after growth of HB101, HB101(pTZ120) and HB101 (pTZ121) were purified using the ion exchange and immunoaffinity chromatography method of MacDonald et al., Meth. Enzymol., 110:347-358 (1985). HPLC and RIA (MacDonald et al., 1982) of these samples, shown in FIG. 2, indicated that HB101(pTZ120) and HB101(pTZ121) produced levels of trans-zeatin comparable to those seen previously in culture filtrates of A. tumefaciens C58 (Regier et al., 1982) (340 and 209 ng/l for HB101(pTZ120) and HB101(pTZ121), respectively). HB101 and (pUC8) did not produce trans-zeatin. Trans-ribosylzeatin and ribosyldihydrozeatin were also produced by HB101(pTZ120) though at much lower levels.

Clearly, production of trans-zeatin by HB101(pTZ120) is associated with the presence of tzs. Trans-zeatin production by HB101(pTZ120), in which the putative reading frame has the same orientation as the lac promoter in the vector, was 60 percent greater than that by HB101(pTZ121).

tzs is Similar to tmr, but is Different in Sequence, Condon Utilization and Function.

There is over 50 percent sequence homology between tmr and tzs. The nucleotide sequence of tzs was determined by first constructing a cleavage site map of the 1.4 kb fragment using various site-specific endonucleases. Overlapping subfragments were cloned in M13 mp18 and mp19, referenced in Yanisch-Perron et al., Gene 33:103-119 (1985). The subfragments were then sequenced by the dideoxynucleoside triphosphate chain termination technique of Sanger et al., Proc. Natl. Acad. Sci. USA, 71:5463-5467 (1977), as modified for use with [$^{35}$S]-dATP by Biggin et al., Proc. Natl. Acad. Sci. USA, 80:3963-3965 (1983). The M13 15-mer sequencing primer and Klenow fragment of DNA polymerase I were from Bethesda Research Laboratories (BRL). The sequencing strategy and the resulting sequence are shown in FIGS. 3 and 4, respectively.

Translation start and stop codons in both strands of the sequence were identified, revealing an open reading frame 729 bp in length which would be expected to code for a polypeptide of 243 amino acids having a molecular weight of 27,598. Maxicell expression of tzs confirmed the production of such a peptide. E. coli strain CSR603 (E. coli Genetic Stock Center, New Haven, CT) was transformed with pTZ120, pTZ121, or pUC19 and expressed as described by the method of Sancar et al., J. Bacteriol., 137:692-693 (1979). In order to maximize expression, cells were starved of sulfate for 1.5 h and then fed with $^{35}S$-Met (New England Nuclear) for 1.5 h. Maxicell experiments showed that the tzs gene product had a $M_r$ of 26,800 dal., consistent with the open reading frame.

FIGS. 5 and 6 show the substantial nucleotide sequence homology and amino acid sequence homology between tmr and tzs. There are also substantial differences as shown in the codon utilization frequency summary of Table I:

TABLE I. Codon Utilization Frequency of tzs and tmr

| First Base | Second Base U | C | A | G | Third Base |
|---|---|---|---|---|---|
| U | 6(2)* | 0(1) | 3(6) | 1(1) | U |
| | 1(7) | 2(1) | 2(1) | 5(3) | C |
| | 1(1) | 1(1) | — | — | A |
| | 8(6) | 7(2) | — | — | G |
| C | 6(9) | 5(3) | 5(9) | 5(2) | U |
| | 11(2) | 2(3) | 2(2) | 6(2) | C |
| | 1(3) | 1(3) | 7(6) | 4(3) | A |
| | 1(6) | 5(2) | 4(10) | 5(3) | G |
| A | 6(9) | 1(1) | 3(3) | 2(1) | U |
| | 8(7) | 3(4) | 1(5) | 3(3) | C |
| | 4(1) | 1(3) | 3(4) | 3(1) | A |
| | — | 4(5) | 3(6) | 0(2) | G |
| G | 3(3) | 4(5) | 10(6) | 3(4) | U |
| | 2(3) | 7(5) | 4(4) | 4(2) | C |
| | 1(0) | 5(9) | 12(6) | 4(9) | A |
| | 3(3) | 6(4) | 9(9) | 4(3) | G |

*Values in parentheses are for tmr.

Like tmr, tzs has functional DMA transferase activity. The extensive sequence homology between tmr and tzs suggested that tzs might have DMA transferase activity. In order to determine whether this was so, tmr was excised (as described below) from pTiAch5 and cloned into pUC18 to give pIP192. DMA

transferase, as decribed in Hommes et al., Meth. Enzymol., 110:340-347 (1985) was assayed in E. coli bearing pIP192, pTZ120 and pTZ121. Simultaneously, cytokinin production by these strains was measured. Table II illustrates the results:

TABLE II. DMA Transferase and Zeatin Production by Recombinant Strains.

| Strain | Insert | DMA Transferase* | Zeatin (ng/l) |
|---|---|---|---|
| HB101(pIP192) | tmr | 64.0 | 35 |
| HB101(pTZ120) | tzs | 143.0 | 19,900 |
| HB101 | none | 9.8 | 11 |

*fmole iP+iPA/g/hr

All strains exhibited significant DMA transferase activity in vitro, but pTZ120 and pTZ121 effectively secreted zeatin, whereas pIP192 did not. The tzs gene is therefore similar to tmr in that it specifies a polypeptide with DMA transferase activity but it differs in that it appears to be designed to function effectively in a prokaryotic environment.

When tzs and tmr are Both Present in E. coli, Zeatin Production is Elevated Substantially Over that Induced by tzs Alone.

In order to attempt to maximize zeatin production, it was decided to introduce both tmr and tzs together into E. coli. Tmr was therefore isolated from pTiAch5 and cloned to give pIP192. Separately, tmr was introduced into pTZ120 to give a new recombinant, pCK101.

pIP192 was constructed in two stages by cloning tmr from pTiAch5 according to cloning strategy outlined in FIG. 7.

The plasmid pTiAch5 was prepared by growing *Agrobacterium tumefaciens* strain Ach5 overnight at 26°C in LBMG medium:

LBMG Composition (per 2 liters)

| | |
|---|---|
| Bactotryptone | 10 g |
| Bacto-yeast extract | 5 g |
| NaCl | 10 g |
| Mannitol | 10 g |
| Glutamic acid | 3 g |
| $K_2HPO_4$ | 0.5 g |
| $MgSO_4.7H_2O$ | 0.2 g |
| pH 6.8 | |

The cells were harvested at $A_{600}$=0.25 and suspended in TE buffer (Tris 50 mM, EDTA 20 mM; pH 8.0, 250 ml). Pronase (5 mg/ml pre-incubated in TE for 1 hour at 37°C, 30 ml) and SDS (20 percent w/v in TE, 15 ml) were added and the cells then incubated at 37°C for 1 hour. The pH was then adjusted to 12.45 (using 3 M NaOH, ca. 11 ml). After 25 min., it was re-adjusted to pH 8.4 by the addition of 2 M Tris-chloride, pH 7.0 (40 ml). 5 M NaCl was added to bring the final NaCl concentration to 1 M, and, after 4 hours at 4°C, the medium was centrifuged at 5000 rpm. The supernatant was then made 10 percent with respect to polyethylene glycol by the addition of 50 percent polyethylene glycol in TE. After incubation at 4°C overnight, the medium was centrifuged at 7000 rpm. The pellet was then resuspended in TE (4 ml). After 1 hour at room temperature, undissolved material was removed by centrifugation at 5000 rpm for 5 min. The Ti plasmid was then purified by equilibrium centrifugation on a caesium chloride/ethidium bromide gradient. Ethidium bromide was added to 0.017 percent, and solid cesium chloride to bring the density to 1.600. Insoluble material was removed by centrifugation at 8500 rpm

for 10 min. and then centrifugation continued at 45000 rpm for 22 hours at 18°C in a Sorvall TV865 rotor. The Ti plasmid was removed by puncturing the side of the tube, dialyzed extensively against TE containing 50 mM NaCl, and then precipitated with ethanol.

By digestion with EcoRI, the tmr-bearing EcoRI fragment 7 was isolated from the *A. tumefaciens* Ti plasmid pTiAch5, identified in Barker et al., *Plant Mol. Biol.*, 2:335-350 (1983). Restriction with EcoRI was carried out in the following mixture:

| | |
|---|---|
| Ti plasmid (5 μg) | 50 μl |
| 10X EcoRI buffer | 10 μl |
| EcoRI (100 units) | 10 μl |
| Water 30 μl | |

The mixture was incubated at 37°C for 4 hours. The digest was then electrophoretically fractionated on a 0.7 percent agarose gel at 150V for 3 hours. After staining with ethidium bromide for 20 min., bands were visualized by ultraviolet transillumination and the desired band (EcoRI-7, 7300 bp) excised together with contaminating 7350 and 7800 bp fragments which ran with it. Because the other fragments did not have internal KpnI sites, they were eliminated during the subsequent cloning procedure.

In order to clone the tmr-containing fragment, the mixed EcoRI fragments, after recovery from the agarose gel, were first digested with KpnI and then ligated to linearized pUC18. The mixed insert bands were digested at 37°C for 3 hours with KpnI using:

| | |
|---|---|
| EcoRI fragment DNA (0.062 pmole) | 4.5 μl |
| 10X KpnI buffer | 1 μl |
| KpnI (10 units) | 1 μl |
| Water | 4 μl |

After incubation, the mixture was treated with phenol and ether, and precipitated with ethanol (2 vol).

Purified pUC18 (5 μg) was sequentially digested at 37°C for 4 hours with KpnI and EcoRI using:

| | |
|---|---|
| pUC18 DNA (5 μg) | 0.5 μl |
| 10X KpnI buffer (BRL) | 1 μl |
| KpnI (10 units) | 1 μl |
| Water | 7.5 μl |

KpnI was then inactivated by heating at 65°C for 10 min. Buffer concentrations were adjusted to those required for EcoRI by addition of 3.4 μl of 1 M Tris (pH 7.5), 2.9 μl of 0.1 M $MgCl_2$, 1.69 μl of 1 M NaCl and 15 μl of $H_2O$. Next, 2 μl of EcoRI (20 units, BRL) was added and the resulting mixture incubated at 37°C for 3 hours. The linearized plasmid was purified on 0.7 percent agarose, electroeluted, treated with phenol then ether, and precipitated with ethanol (2 vol).

The insert was ligated to the EcoRI- and Kpn-cut pUC18 (molar ratio, 2:1) by incubation at 16°C for 16 hours using the mixture:

| | |
|---|---|
| Insert DNA (0.0615 pmole) | 4.5 μl |
| Vector DNA (0.031 pmole) | 2.3 μl |
| 10X Ligase buffer | 1.0 μl |
| T4 DNA Ligase (1.5 units) | 0.5 μl |
| Water | 1.7 μl |

The resulting intermediate plasmid pAR13 was transformed to HB101, activated with $CaCl_2$ as described in Silhavy et al., Experiments with Gene Fusions, Cold Spring Harbor Laboratory (New York, 1984), pp. 169-170. Transformants were isolated on LB plates containing 50 μg/ml of ampicillin.

The mini-lysate procedure of Holmes et al., Anal. Biochem 114:193 (1981) was used to isolate recombinant plasmid DNA from individual transformants. Each was then characterized

by double digestion with EcoRI and KpnI, using conditions outlined above. A successful ligation provided fragments derived from the insert (4200 bp of DNA derived from pTiAch5 EcoRI fragment 7), (see Fig. 7) and from the vector (2700 Kbp). pAR13 has such characteristics.

This intermediate plasmid, pAR13, served as the source of both pIP192 and PCK101 as described below.

In order to construct pIP192, pAR13 DNA was prepared from HB101(pAR13) by the method of Birnboim et al., Nucleic Acids Res., 7:1513-1523 (1979). A tmr-containing fragment was isolated from pAR13 by first digesting it at 37°C for 5 hours with NdeI (which cuts 411 bp to the left of the tmr coding region itself, see Fig. 7) The digestion mixture included:

| | |
|---|---|
| pAR13 DNA (22 μg) | 5 μg |
| 10X Core buffer (BRL) | 1 μl |
| NdeI (20 units) | 4 μl |
| Water | 27 μl |

After phenol extraction and precipitation, the NdeI cut ends were blunted with the Klenow fragment of DNA polymerase I:

| | |
|---|---|
| Linearized pAR13 DNA (20 μg) | 15 μl |
| Deoxynucleotide triphosphates (2mM) | 0.5 μl |
| 10X buffer | 2 μl |
| Klenow fragment DNA polymerase I (3 units) | 1.7 μl |
| Water | 0.8 μl |

The reaction was terminated after 20 min. at room temperature by addition of phenol and precipitate the product with ethanol at -20°C.

The DNA was recut with KpnI in order to excise the desired insert. Digestion was at 37°C for 5 hours using a reaction mixture which contained:

0248984

| | |
|---|---|
| Blunted pAR13 DNA (2 pmole) | 10 µl |
| 10X KpnI buffer | 11 µl |
| KpnI (10 units) | 11 µl |
| Water | 78 µl |

The KpnI - blunt end (1478 bp) fragment was isolated by preparative gel electrophoresis on 0.7 percent agarose, purified by phenol treatment and precipitated with ethanol.

Finally, the KpnI-cut fragment was ligated into HincII-cut, KpnI-cut pUC19 to give pIP192. The plasmid pUC19 was prepared from a culture of HB101(pUC19) as identified by Birnboim et al. in Nucleic Acids Res., 7:1513-1523 (1979), and linearized it with SmaI. Digestion conditions consisted of incubation at 30°C for 2 hours of:

| | |
|---|---|
| Plasmid DNA (5 µg) | 1 µl |
| 10X buffer | 1 µl |
| SmaI (10 units) | 2 µl |
| Water | 6.5 µl |

Subsequently, 2 µl of 10X KpnI buffer, 1 µl of KpnI (9 units) and 17 µl of water were added and incubation continued for a further 2 hours at 37°C.

The linearized vector was separated from small DNA fragments on 0.7 percent agarose, purified by phenol treatment and ethanol precipitation and ligated to the insert at an insert to vector ratio of 1.5:1. Specifically, the following mixture was incubated at 16°C for 18 hours:

| | |
|---|---|
| Insert DNA (72 ng) | 3.0 µl |
| pUC19 DNA (88 ng) | 0.3 µl |
| 10X Ligase buffer | 1.0 µl |
| T4 Ligase (1.5 units) | 1.0 µl |

This strategy preserved the correct orientation of tmr with respect to the lac Z promoter of pUC19. The insert can only be ligated to pUC19 in one orientation because of the presence of sticky (KpnI) and blunt (Nde/Klenow) ends.

The resulting pIP192 plasmids can be characterized by digestion with restriction enzymes. The insert has an internal PstI site as described by Barker et al., Plant Molecular Biol., 2:335-350 (1983). Digestion with KpnI, HindIII, and PstI should produce insert fragments of 627 and 851 bp.

The pIP192 plasmid was transformed to HB101. HB101(pIP192) transformants were selected on LB-ampicillin plates.

Next, a tmr-containing fragment was inserted into pTZ120 to complete the construction of pCK101. The tmr-containing fragment from pAR13 was isolated (by the procedure described above for construction of pIP192). First, pAR13 is cut with NdeI, and then cut with HindIII in the vector just to the right of the insert-vector border. This fragment was then blunted at both ends by treatment with Klenow polymerase. The blunt-ended fragment was then ligated into SmaI-cut and alkaline phosphatase-treated pTZ120. In detail, the procedure was to digest pAR13 at 37°C for 9 hours with HindIII using the reaction mixture:

| | |
|---|---|
| pAR13 DNA (32 µg) | 7 µl |
| 10X Core buffer (BRL) | 8 µl |
| HindIII (80 units) | 8 µl |
| Water | 57 µl |

The buffer concentrations were then adjusted to those needed by NdeI by addition of 3.2 µl of Tris (1 M, pH 7.6), 2.2 µl of NaCl (5 M), 0.75 µl of DTT (400 mM), 6µl $H_2O$, and 24 units (8 µl) of NdeI. Final reaction volume was 100 µl. Incubation took place at 37°C for 13 hours.

After digestion, the tmr-containing insert (1520 bp) was gel-purified and the ends blunted by treatment with Klenow polymerase, using:

| | |
|---|---|
| Insert DNA (5 µg) | 16.7 µl |
| Deoxynucleotide triphosphates (2 mM) | 0.5 µl |
| 10X buffer | 2 µl |
| Klenow DNA polymerase (3.6 units) | 0.8 µl |

Incubation was at 25°C for 25 min.

Next, pTZ120 was digested with SmaI in the mixture:

| | |
|---|---|
| pTZ120 DNA (25.5 µg) | 10 µl |
| 10X buffer | 5 µl |
| SmaI (25 units) | 5 µl |
| Water | 30 µl |

Incubation was at 37°C fo 8 hours.

Bacterial alkaline phosphatase (1200 units) was added to dephosphorylate the vector ends and incubate at 65°C for 1 hour. Excess bacterial alkaline phosphatase was eliminated by treatment with proteinase K (2 µl of 10 mg/ml were added and incubated at 37°C for 30 min.). Finally, the linearized vector was purified by extraction with phenol and ethanol precipitation.

The blunt tmr-containing insert was ligated to the vector at an insert:vector ratio of 1:1. The reaction mixture was:

| | |
|---|---|
| Insert DNA (55 ng) | 5.0 µl |
| pTZ120 DNA (149 ng) | 1.6 µl |
| 10X Ligase buffer | 1.0 µl |
| T4 Ligase (1.8 units) | 1.5 µl |
| Water | 0.9 µl |

Incubation was at 16°C for 19 hours. The resulting plasmid was transformed to HB101 and transformants selected on LB-ampicillin plates.

Because the insert was blunt at both ends, HB101 transformants contained plasmids with tmr in either orientation. It was therefore necessary to select only those in which the tmr orientation was correct with respect to the lac-Z promoter (Fig. 8). If tmr was in the correct orientation with respect to the lac-Z promoter, double digestion with EcoRI and KpnI produced two fragments (1500 and 996 bp). If the insert had the opposite orientation, two fragments were generated, but were 2474 bp and 40 bp. Putative pCK101 plasmids were isolated from individual transformants, digested with EcoRI and KpnI and those having the correct orientation selected. The transformant in which tmr was in an orientation appropriate for its expression to be mediated by the lac promoter from pTZ120, was designated HB101(pCK101). It contains the pCK101 plasmid illustrated in FIG. 8.

The following biologically pure cultures of Escherichia coli were deposited on January 24, 1986, and are available from the permanent collection of the American Type Culture Collection, Rockville, Maryland, U.S.A.: HB101(pTZ120), ATCC 53437; HB101(pIP192), ATCC 53436; and HB101(pCK101), ATCC 53438.

To measure the amount of cytokinins produced, HB101(pCK101) was grown to mid-log phase and zeatin production was estimated by HPLC and RIA. The data are shown in Table III. Zeatin production from HB101(pCK101) was much higher than that of HB101 derivatives bearing either tzs (pTZ120, pTZ121) or tmr (pIP192) alone. This increase in zeatin production occurred even without the use of high expression promoters.

TABLE III. Cytokinin Production by Recombinant Strains Carrying tmr or tzs.

| Strain | Insert | Zeatin* | iP* |
|---|---|---|---|
| HB101(pTZ120) | tzs | 19,900 | 8,700 |
| HB101(pIP192) | tmr | 35 | 197 |
| HB101(pCK101) | tmr + tzs | 110,000 | 14,900 |
| HB101 | none | 11 (trace) | 180 |

*ng/l mid log phase

II. PTZ

P. savastanoi Secretes Zeatin, Ribosylzeatin and Their 1"-methyl Derivatives

Cytokinin production by strains of the phytopathogenic bacterium Pseudomonas syringae pv. savastanoi (P. savastanoi) was measured by immunoaffinity chromatography of culture medium on immobilized anti-cytokinin antibodies, followed by high performance liquid chromatography, radioimmunoassay and mass spectrometry.

The tested strains of P. savastanoi produced zeatin at very high levels, and one strain also produced high levels of ribosyl-1"-methylzeatin. Genes specifying cytokinin production were found to be carried on P. savastanoi plasmids and were cloned and expressed in E. coli.

Bacterial Strains and Growth

P. savastanoi strains PB213-2, PB1050, and EW1006 have been described previously [Comai et al., J. Gen. Microbiol. 128:2157-2163 (1982)]. P. myricae isolates (7118 and 7120) were obtained from the Plant Diseases Division Culture Collection, New Zealand Department of Scientific and Industrial Research. Both sets were grown in King-B medium [King et al., J. Lab. Clin. Med. 44:301-307 (1954)] or Miller's Minimal A medium [Miller, Experiments in Molecular Genetics (Cold Spring Harbor, NY, p. 432, 1972) containing 0.2 percent (w/v) glucose

and 0.05 percent (w/v) casamino acids. Since strains readily lost plasmids by segregation, stocks were maintained in the presence of 50 percent (v/v) glycerol at -70°C. For cytokinin analysis, cultures were grown at 25°C in silanized glassware and harvested at mid-log phase ($A_{600}$ = 0.6). Prior to analysis, the number and size of the plasmids was determined.

Plasmid Isolation

Plasmid DNA was isolated by modification of the high temperature alkaline lysis procedure of Kado and Liu [Kado et al., J. Bacteriol. 145:1365-1373 (1981)]. Cultures were grown overnight in King-B broth, aliquots (0.75 ml) were harvested by centrifugation at 15,000 x g for 5 min., and the cells were resuspended in 0.1 ml of lysis buffer (50 mM Tris, 3 percent SDS (v/v); pH 12.6) at 60°C for 45 min. Sodium chloride (5 M) was added to a final concentration of 0.5 M, and the solution was shaken with an equal volume of phenol:chloroform (1:1, v/v). The two phases were separated by centrifugation and the aqueous phase was analyzed directly by gel electrophoresis.

Radioimmunoassay

Direct RIA of culture filtrates was carried out as described previously [MacDonald E. M. S., Akiyoshi, D. E. and R. O. Morris, J. Chromatog. 214:101-109 (1982)]. Cells were grown overnight to late log phase, and the supernatants were harvested by centrifugation at 15,000 x g for 5 min. Radioimmunoassay was performed directly on aliquots (0.35 ml) of supernatant using monoclonal anti-tZR antibody and [$^3$H]zeatin trialcohol (5 nCi (1 Ci = 37 GBq); 2.7 Ci/mmol). Estimation of the amount of total zeatin-like material present was made by comparison with standards assayed under identical conditions.

## Immunoaffinity, HPLC, RIA and Mass Spectrometry of Cytokinins

Aliquots (0.5, 1 of 100 ml) of mid log phase culture filtrates were passed sequentially through connected columns of DEAE cellulose (DE-52, 2 ml or 20 ml) and an immunoaffinity matrix [MacDonald, E. M. S. and R. O. Morris in Methods in Enzymology 110:347-358 (1985)] comprised of a mixture of 1.5 ml of anti-tZR antibody-cellulose and 1 ml of anti-iPA antibody-cellulose equilibrated with 40 mM ammonium acetate buffer, pH 6.5 (Buffer A, 2 vol). The capacity of such columns was approximately 113 ng of ribosylzeatin and 36 ng of *iso*-pentenyladenosine. Samples were applied in a total volume of 3 ml (diluted with buffer A); the columns were washed with buffer A (10 vol); and the DE-52 column was then discarded. The immunoaffinity column was washed with buffer A (5 vol), buffer A containing 0.5 M NaCl and 2 percent v/v DMSO (2.5 vol), buffer A (5 vol), and the cytokinins were eluted with 3 ml of methanol. Eluates were evaporated to dryness *in vacuo* and dissolved in a methanol:triethylamine acetate buffer, pH 3.35 (1:1, v/v) for HPLC.

HPLC and RIA was performed as described previously [MacDonald, E. M. S. and R. O. Morris in Methods in Enzymology 110:347-358 (1985)]. Recoveries were measured in replicate samples which contained [$^3$H]ribosylzeatin or [$^3$H]*iso*-pentenyladenosine (5 Ci/mmol). Cytokinin levels expressed in the text have been corrected for recovery (usually 67-71 percent). Individual HPLC fractions were evaporated to dryness *in vacuo* and permethylated for mass spectrometry as described previously [Morris, R. O., Plant Physiol. 59:1029-1033 (1977)]. Spectra were acquired on a Finnegan 402 quadrupole mass spectrometer at an electron energy of 35 eV.

Cytokinin Production by P. savastanoi.

Preliminary RIA of unfractionated culture filtrates from a number of virulent P. savastanoi (PB1050, EW1006, and PB213) and P. myricae (7118 and 7120) isolates indicated that they contained approximately 1000 times more zeatin-like cytokinins than filtrates from A. tumefaciens strain C58 [Regier, D. A. and R. O. Morris, Biochem. Biophys. Res. Comm. 104:1560-1566 (1982)]. Two strains were selected for further study: EW1006 (isolated from olive knot) and PB213-2, a deletion mutant of PB213, (originally isolated from oleander knot) that has lost part of the pIAA1 plasmid and which no longer secretes IAA [Comai et al., J. Gen. Microbiol. 128:2157-2163 (1982)]. P. savastanoi strain PB213-2 secretes zeatin (80 ng/ml) and ribosylzeatin (80 ng/ml). Even higher levels of zeatin (400 ng/ml) are produced by the olive-specific strain EW1006, which also produces 180 ng/ml of the recently identified cytokinin, ribosyl-1"-methylzeatin.

In order to identify the cytokinins and to estimate the levels present, aliquots of culture filtrates from both strains were applied to immunoaffinity columns containing immobilized mixtures of monoclonal antibodies raised against trans-ribosylzeatin and iso-pentenyladenosine [MacDonald, E. M. S. and R. O. Morris in Methods in Enzymology 110:347-358 (1985)]. Following elution, the purified cytokinin mixtures were analyzed by HPLC and RIA.

PB213-2 had major UV-absorbing peaks, eluting at 15.2 min. and 19.8 min., that cross-reacted with anti-tZR antibody. Retention times of these peaks were identical to those of trans-zeatin and trans-ribosylzeatin. A much smaller peak at 10.2 min. also cross-reacted with anti-tZR antibody. Its retention time was identical to that of zeatin-9-glucoside, but it was not characterized further. A fourth peak at 33.5 min. cross-reacted with anti-iPA antibody.

Cytokinins were also present in culture filtrates of EW1006. The $A_{254}$ trace displayed few peaks (attesting to the efficacy of the immunoaffinity purification), of which four cross-reacted with anti-tZR antibody and one with anti-iPA antibody. Those cross-reacting with anti-tZR antibody had retention times corresponding to zeatin (14.5 min.), dihydrozeatin (16.5 min.), ribosylzeatin (18.8 min.) and ribosyldihydrozeatin (20.6 min.). Small but significant amounts of iso-pentenyladenosine were detected at 33.3 min.

In addition to known cytokinins, EW1006 filtrates contained a substantial amount of a UV-absorbing substance (Peak I) at 25.9 min. which cross-reacted weakly with anti-tZR antibody. Its retention time on HPLC was indicative of a polarity intermediate between that of ribosylzeatin and iso-pentenyladenosine which would be consistent with the properties of the novel cytokinin ribosyl-1"-methylzeatin recently identified by Surico et al. [Surico et al., Phytochem. 24:1499-1502 (1985)].

Confirmation of Cytokinin Identity by Mass Spectrometry

In order to confirm the presence of zeatin and ribosylzeatin, and determine the identity of Peak I, appropriate HPLC fractions were permethylated and examined by mass spectrometry. Permethylation of the zeatin fractions from PB213-2 of EW1006 gave derivatives whose mass spectral properties were identical to those of authentic permethylzeatin [Morris, Plant Physiol. 59:1029-1033 (1977)]. All major ions were present at the expected intensities, including the molecular ion at m/z = 261 and fragment ions at m/z = 230, 216,

199, 174, 162, 164 and 134. Retention times of zeatin on HPLC and of permethylzeatin on GLC (data not shown), were identical to those of standards.

Permethylation of the ribosylzeatin fraction from PB213-2 gave a product whose mass spectrum (FIG. 9A) was identical to that of authentic permethylribosylzeatin. The molecular ion and prominent fragment ions were present at m/z = 421, 390, 348, 246, 230, 216 and 174. HPLC and GLC retention times were identical to those of trans-ribosylzeatin or its permethyl derivative.

Permethylation and perdeuteromethylation of Peak I from EW1006 gave the spectra shown in FIG. 9B and C. There was an obvious molecular ion at m/z = 435 and fragment ions at m/z = 404, 390, 362, 286, 260, 246, 230 and 188. The spectrum was almost identical to that of permethylribosylzeatin (FIG. 9A) except that clearly homologous fragment ions in FIG. 9A were increased in mass by 14 amu over those in FIG. 9B, indicating the presence of an extra methyl group. The position of this extra methyl group could be determined by examination of the fragmentation pattern. The Peak I fragment ion at m/z 188 (FIG. 9B) corresponds to the ribosylzeatin fragment ion, previously identified, at m/z = 174, which ion is a tricyclic structure [Shannon et al., New Zealand J. Sci. 9:833 (1966)] resulting from side-chain cleavage (with loss of side chain carbons 3, 4 and 5) and loss of ribose. The mass increase of 14 amu indicated tha the extra methyl group must be present either on the ring or on the 1" or 2" positions of the side chain. The mass spectrum of underivatized Peak I (data not shown) contained fragment ions at m/z-148 and 119 which have been assigned to 6-methyleneimino-purinyl and purinyl cations

respectively [Shaw et al., J. Amer. Chem. Soc. 92:2501-2522 (1970)]. Analogous ring-methylated ions were not present, indicating that the methyl group must be attached to the side chain. Peak I appears, therefore, to be ribosyl-1"-methylzeatin as proposed by Surico et al. [Surico et al., Phytochem. 24:1499-1502 (1985)]. The spectrum of the perdeuteromethyl derivative confirmed this structural assignment. The molecular and fragment ion masses increased by the expected amounts in all cases.

## Isolation of the Cytokinin Biosynthetic Genes of P. savastanoi and Expression in E. coli

A number of spontaneous plasmid deletion mutants of P. savastanoi strain EW1006 were isolated (by plasmid screening of randomly selected colonies) and examined (by RIA) for their ability to produce cytokinins. Production of zeatin was abolished by removal of a segment of the larger (105 kb) plasmid present in EW1006. Elimination of the smaller (70 kb) plasmid had no effect on zeatin production.

In order to clone the cytokinin-specifying genes of the 105 kb plasmid of strain EW1006, P. savastanoi plasmid DNA was isolated from 1 g of pelleted cells. Cells were lysed in 100 ml of lysis buffer and extractions were performed as described above. Plasmids were purified by CsCl density gradient centrifugation [Draper et al., Experientia 38:101-102 (1982)]. A partial Sau3A digest of the total plasmid DNA was performed and the resulting fragments were ligated to the wide host range vector pRK290 [Ditta et al., Proc. Natl. Acad. Sci. USA 77:7347-7351 (1980)] which had been cut with BglII and treated

with alkaline phosphatase. The ligation was performed by the procedure of Ditta et al. as described in Proc. Natl. Acad. Sci. USA, 77:7347-7351 (1980)].

E. coli HB101 [$F^-$, hsd S20($r_{B-}$, $m_B^-$), sup E44, ara 14, galK2, lac Y1, pro A2, rps L20 (Smr), xyl 5, mtl 1, rec A13, $\lambda^-$ (Boyer et al., J. Mol. Biol. 41:459-472 (1969)] was transformed with the ligation mixture. $Tet^R$ colonies bearing large (5-20 kb) inserts were selected on LB plates containing tetracycline (25 mg/l) for further examination. One clone, HB101 (pPS001), produced zeatin during growth in liquid culture. Restriction enzyme digestion of pPS001 gave the map illustrated in FIG. 10. The insert is about 14.5 kb.

The cytokinin biosynthetic locus (ptz), was further localized by sequential treatment with restriction enzymes EcoRI, BglII and BamHI, followed by religation to give HB101 (pPS002) which had lost a 3.7 kb EcoRI fragment from pPS001. Further subcloning of the ptz locus was accomplished by excision of a BglII-BamHI fragment, yielding pPS003. These steps eliminated substantial amounts of DNA while retaining the ability to produce zeatin. The EW1006 insert in pPS003 is approximately 4.5 kb in size.

The pPS003 plasmid was transformed to HB101 to provide E. coli HB101 (pPS003).

A biologically pure culture of E. coli HB101 (pPS003), ATCC 53439, was deposited on January 24, 1986, and is available from the permanent collection of the American Type Culture Collection, Rockville, Maryland, U.S.A.

Expression of the Biosynthetic Genes

Expression of pPS003 in E. coli HB101 followed by cytokinin analysis by immunoaffinity chromatography and HPLC indicated that zeatin (9.7 ng/ml) and ribosylzeatin (3.6 ng/ml) were present along with lesser amounts of iPA (2.7 ng/ml) and iP (3.1 ng/ml). Therefore, the insert of pPS003 contains the genes responsible for zeatin synthesis. Estimates of the level of zeatin suggested that about 16 ug/l was being produced. In subsequent work, a new plasmid has been constructed by inserting the promoter and the coding region of ptz in the multi-copy bacterial vector pBR325. Preliminary experiments show expression of ptz under these circumstances to result in production of zeatin and iso-pentenyladenine at levels at least ten times greater than that of pPS003.

DNA Sequence

FIG. 11A shows a restriction map of the 5.2 kb pPS003 insert and, in greater detail, the restriction sites within and flanking the ptz open reading frame (ORF). Coding regions of ptz are shown by thick lines. Direction of transcription is from right to left. Overlapping fragments were sequenced by the strategy outlined in FIG. 10B. Horizontal arrows indicate the direction and extent of DNA segments analyzed.

The nucleotide sequence of the gene is presented in FIG. 12. The deduced amino acid sequence is shown below the nucleotide sequence. Base position +1 is the "A" nucleotide of the start codon. Sequences similar to prokaryotic transcription initiation and termination signals are underlined. The ribosome binding site is boxed. Only one ORF greater than 300

nucleotides was found within the 1.5 kb sequence. This 702 nucleotide ORF encodes a 234 amino acid protein with a predicted subunit molecular weight of 26,816.

Expression of ptz by in vitro translation produces a radiolabeled protein with a $M_r$ of 27,500. Although several other labeled peptides are observed, these are also present in controls lacking added DNA template.

Examination of the sequence upstream from the translation initiation codon (ATG, nucleotide +1) revealed several regions characteristic of prokaryotic regulatory sequences. A hexanucleotide sequence, AGGAGG, located at position -11 to -6 (FIG. 12) precisely matches the consensus Shine-Dalgarno prokaryotic ribosome binding site [Shine et al., Proc. Natl. Acad. Sci. USA 71:1342-1346 (1974) and Gold et al., Ann. Rev. Microbiol. 35:365-403 (1981)]. Centered at nucleotide position -80 is the sequence TATAATG that is identical to the consensus Pribnow box [Pribnow, Proc. Natl. Acad. Sci. USA 72:784-788 (1975)] which is typically located 10 bases upstream from the transcription initiation site. In addition, three regions located at positions -105 (TTGGC), -110 (TTAGT) and -116 (TTGAA) closely resemble the consensus -35 sequence (TTGAC) of prokaryotic promoters [Rosenberg et al., Ann. Rev. Genet. 13:319-353 (1979)]. Downstream from the translation termination codon, beginning at nucleotide position +726, is a 34 bp inverted repeat sequence typical of prokaryotic transcription termination signals [Rosenberg et al., Ann. Rev. Genet. 13:319-353 (1979)].

While we have shown and described preferred embodiments of our invention, it will be apparent that changes may be made without department form the spirit of the invention. Therefore, the scope of the invention should only be determined by the following claims.

The features disclosed in the foregoing description, in the claims and/or in the accompanying drawings may, both separately and in any combination thereof, be material for realising the invention in diverse forms thereof.

The deposits ATCC 53436, 53437, 53438 and 53439 were all made under the Budapest Treaty.

The following clauses define various aspects of the present invention.

1. A recombinant DNA molecule comprising a first DNA segment containing a gene selected from the group consisting of tzs substantially homologous to tzs from an Agrobacterium Ti plasmid and ptz from a P. savastanoi plasmid, the first segment being flanked by second and third DNA segments that are not substantially homologous to the DNA that flanks the first segment in its plasmid of origin.

2. The molecule of clause 1 that is a replicon.

3. The molecule of clause 1 wherein the tzs is substantially homologous to the tzs present in plasmid pTZ120.

4. The molecule of clause 1 wherein the tzs has a sequence substantially the same as that shown in FIG. 4.

5. A recombinant DNA molecule comprising a first DNA segment containing tmr and tzs substantially homologous to tmr and tzs from Agrobacterium Ti plasmids, flanked by second and third DNA segments that are not substantially homologous to the DNA that flanks either the tmr or the tzs in the Ti plasmids.

6. The molecule of clause 5 that is a replicon.

7. The molecule of clause 5 wherein the tmr and tzs are substantially homologous to the tmr and tzs in plasmid pCK101.

8. The molecule of clause 5 wherein the tzs is substantially homologous to the tzs present in plasmid pTZ120.

9. The molecule of clause 5 wherein the tzs has a sequence substantially the same as that shown in FIG. 4.

10. A replicon comprising cloning vector joined with tzs of the type present in plasmid pTZ120.

11. The replicon of clause 10 wherein the joined tzs is substantially homologous to the joined tzs in plasmid pTZ120.

12. The replicon of clause 10 wherein the joined tzs has the sequence shown in FIG. 4.

13. A replicon comprising a cloning vector joined with tmr and tzs of the type present in plasmid pCK101.

14. The replicon of clause 13 wherein the joined tmr and tzs are substantially homologous to the joined tmr and tzs in plasmid pCK101.

15. The replicon of clause 13 wherein the joined tzs is substantially homologous to the joined tzs in plasmid pTZ120.

16. The replicon of clause 13 wherein the joined tzs has the sequence shown in FIG. 4.

17. Plasmid pCK101 which is present in the culture of Escherichia coli HB101 having deposit accession number ATCC 53438.

18. Plasmid pTZ120 which is present in the culture of Escherichia coli HB101 having deposit accession number ATCC 53437.

19. Plasmid pCK101 which comprises plasmid pUC8 joined with tmr and tzs from Agrobacterium tumefaciens and which has the structure shown in FIG. 8.

20. The plasmid of clause 19 in which the tzs has the sequence of FIG. 4.

21. Plasmid pTZ120 which comprises plasmid pUC8 joined with tzs from an Agrobacterium tumefaciens Ti plasmid, the tzs having the sequence shown in FIG. 4.

22. Escherichia coli HB101 containing the plasmid pCK101, having the deposit accession number ATCC 53438.

23. Escherichia coli HB101 containing the plasmid pTZ120, having the deposit accession number ATCC 53437.

24. The molecule of clause 1 wherein the gene material is substantially homologous to the ptz present in plasmid pPS003.

25. The molecule of clause1 wherein the gene material is characterized by a restriction endonuclease cleavage map as shown in FIG. 11.

26. The molecule of clause1 wherein the gene material has a sequence substantially homologous to that shown in FIG. 12.

27. A replicon comprising cloning vector joined with cytokinin biosynthetic gene material of the type present in plasmid pPS003.

28. A replicon of clause27 wherein the joined gene material is substantially homologous to that found in the joined ptz in plasmid pPS003.

29. A replicon of clause27 wherein the gene material is characterized by a restriction endonuclease cleavage map as shown in FIG. 11.

30. A replicon of clause27 wherein the gene material has a sequence substantially the same as that shown in FIG. 12.

31. Plasmid pPS003 which comprises plasmid pRK290 joined with cytokinin biosynthetic gene material from *P. savastanoi*.

32. The plasmid of clause 31in which the joined gene material is characterized by a restriction endonuclease cleavage map as shown in FIG. 11.

33. The plasmid of clause 31 in which the joined gene material has a sequence substantially the same as that shown in FIG. 12.

34. Plasmid pPS003 which is present in the culture of E. coli HB101 having deposit accession number ATCC 53439.

35. E. coli HB101 containing the plasmid pPS003, having the deposit accession number ATCC 53439.

CLAIMS

1. A recombinant DNA molecule comprising a first DNA segment containing a gene selected from the group consisting of tzs substantially homologous to tzs from an *Agrobacterium* Ti plasmid and ptz from a *P. savastanoi* plasmid, the first segment being flanked by second and third DNA segments that are not substantially homologous to the DNA that flanks the first segment in its plasmid of origin.

2. A recombinant DNA molecule comprising a first DNA segment containing tmr and tzs substantially homologous to tmr and tzs from *Agrobacterium* Ti plasmids, flanked by second and third DNA segments that are not substantially homologous to the DNA that flanks either the tmr or the tzs in the Ti plasmids.

3. A replicon comprising cloning vector joined with tzs of the type present in plasmid pTZ120.

4. A replicon comprising a cloning vector joined with tmr and tzs of the type present in plasmid pCK101.

5. Plasmid pCK101 which is present in the culture of *Escherichia coli* HB101 having deposit accession number ATCC 53438.

6. Plamid pTZ120 which is present in the culture of *Escherichia coli* HB101 having deposit accession number ATCC 53437.

7. Plasmid pCK101 which comprises pUC8 joined with tmr and tzs from *Agrobacterium tumefaciens* and which has the structure shown in FIG. 8.

8. Plasmid pTZ120 which comprises plasmid pUC8 joined with tzs from an *Agrobacterium tumefaciens* Ti plasmid, the tzs having the sequence shown in FIG. 4.

9. *Escherichia coli* HB101 containing the plasmid pCK101, having the deposit accession number ATCC 53438.

10. *Escherichia coli* HB101 containing the plasmid pTZ120, having the deposit accession number ATCC 53437.

11. A replicon comprising cloning vector joined with cytokinin biosynthetic gene material of the type present in plasmid pPS003.

12. Plasmid pPS003 which comprises plasmid pRK290 joined with cytokining biosynthetic gene material from *P.savastanoi*.

13. Plasmid pPS003 which is present in the culture of *E.coli* HB101 having deposit accession number ATCC 53439.

14. *E.coli* HB101 containing the plasmid pPS003, having the deposit accession number ATCC 53439.

FIG. 1A

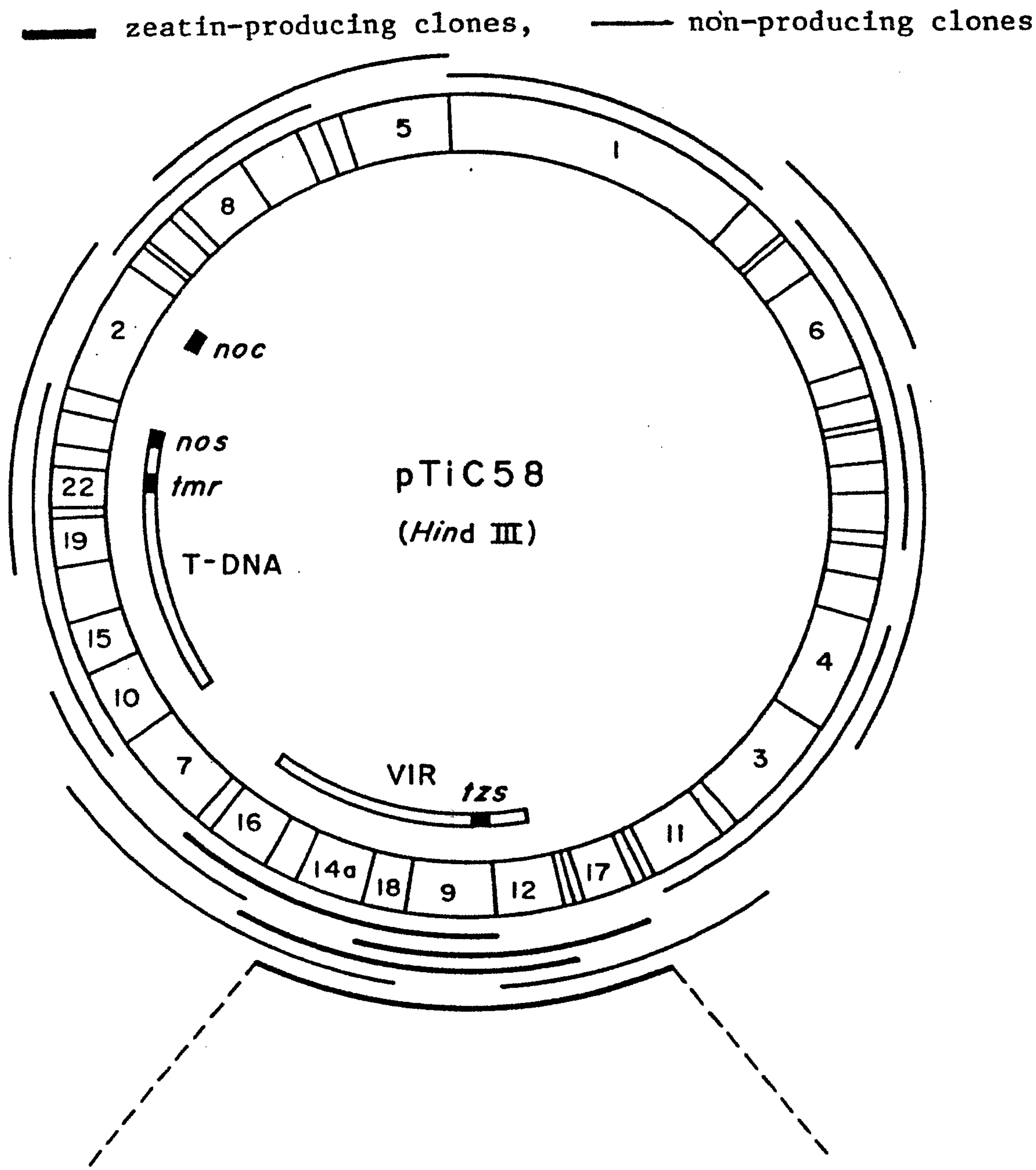


0248984

1/16

15.07.87

FIG. 1B

Subcloning strategy.

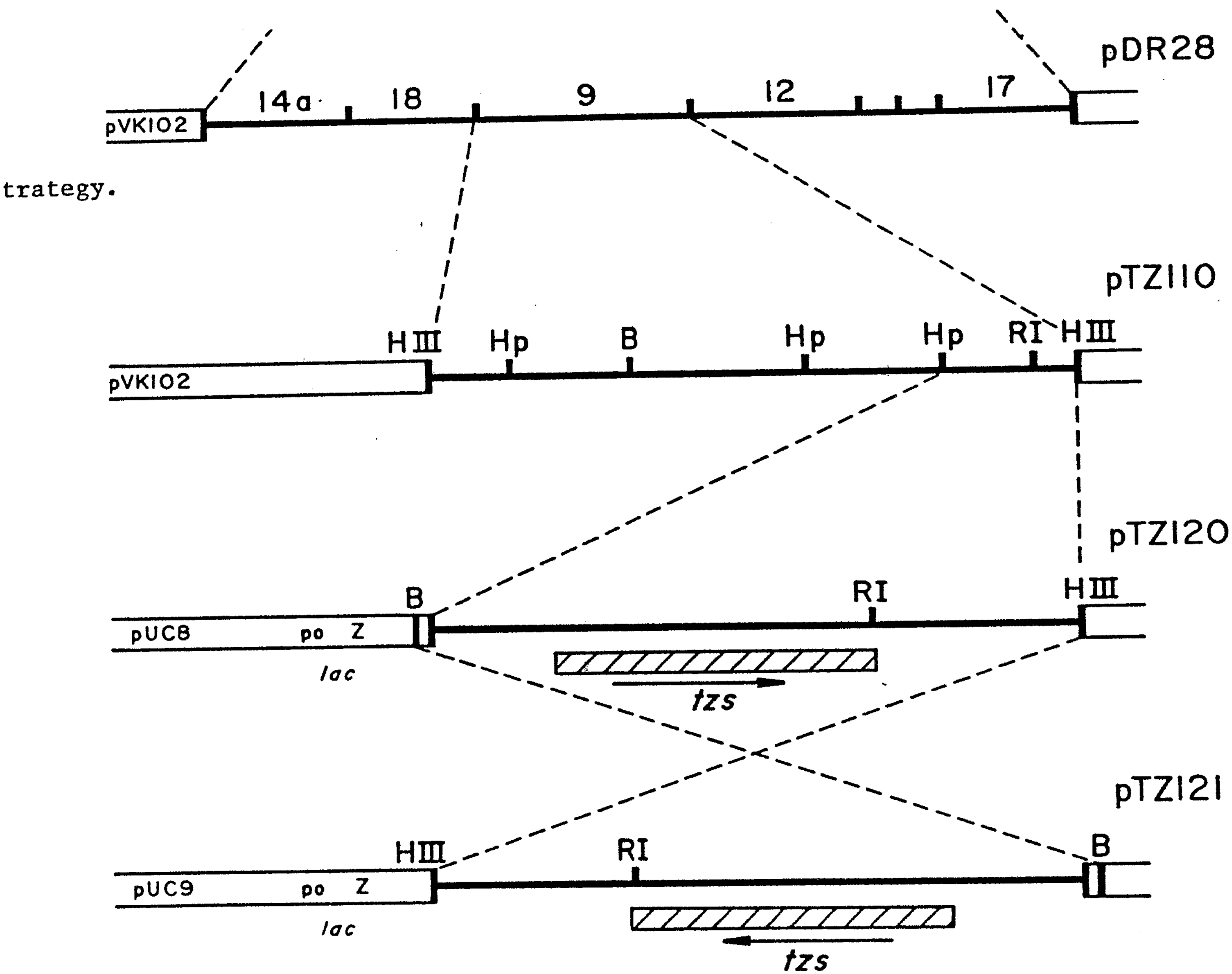


Isolation and subcloning of tzs from pTiC58

FIG. 2A

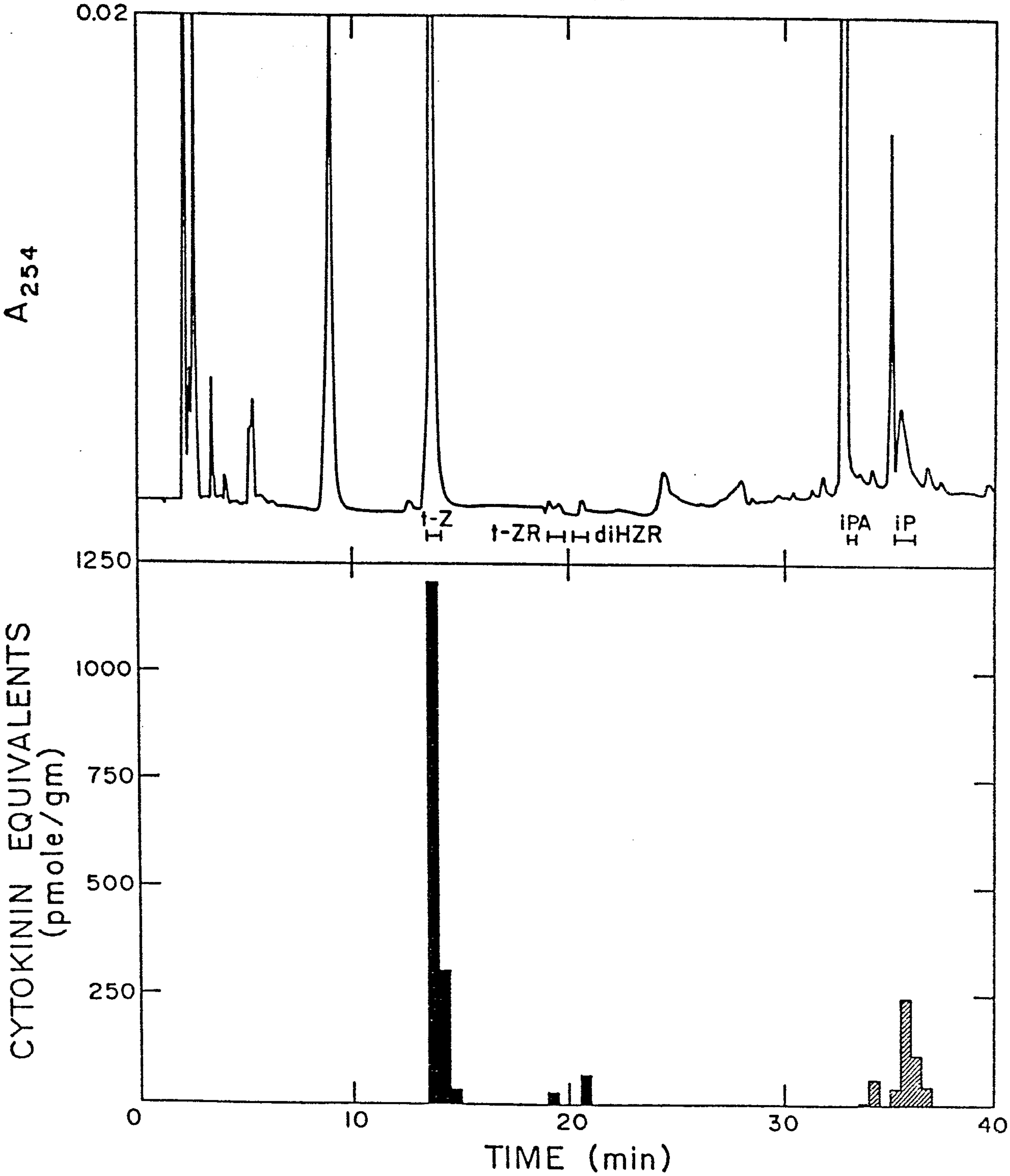
0.02
A254
t-Z
t-ZR
diHZR
iPA
iP
1250
1000
750
500
250
CYTOKININ EQUIVALENTS (pmole/gm)
0
10
20
30
40
TIME (min)

FIG. 2B

FIG. 2C

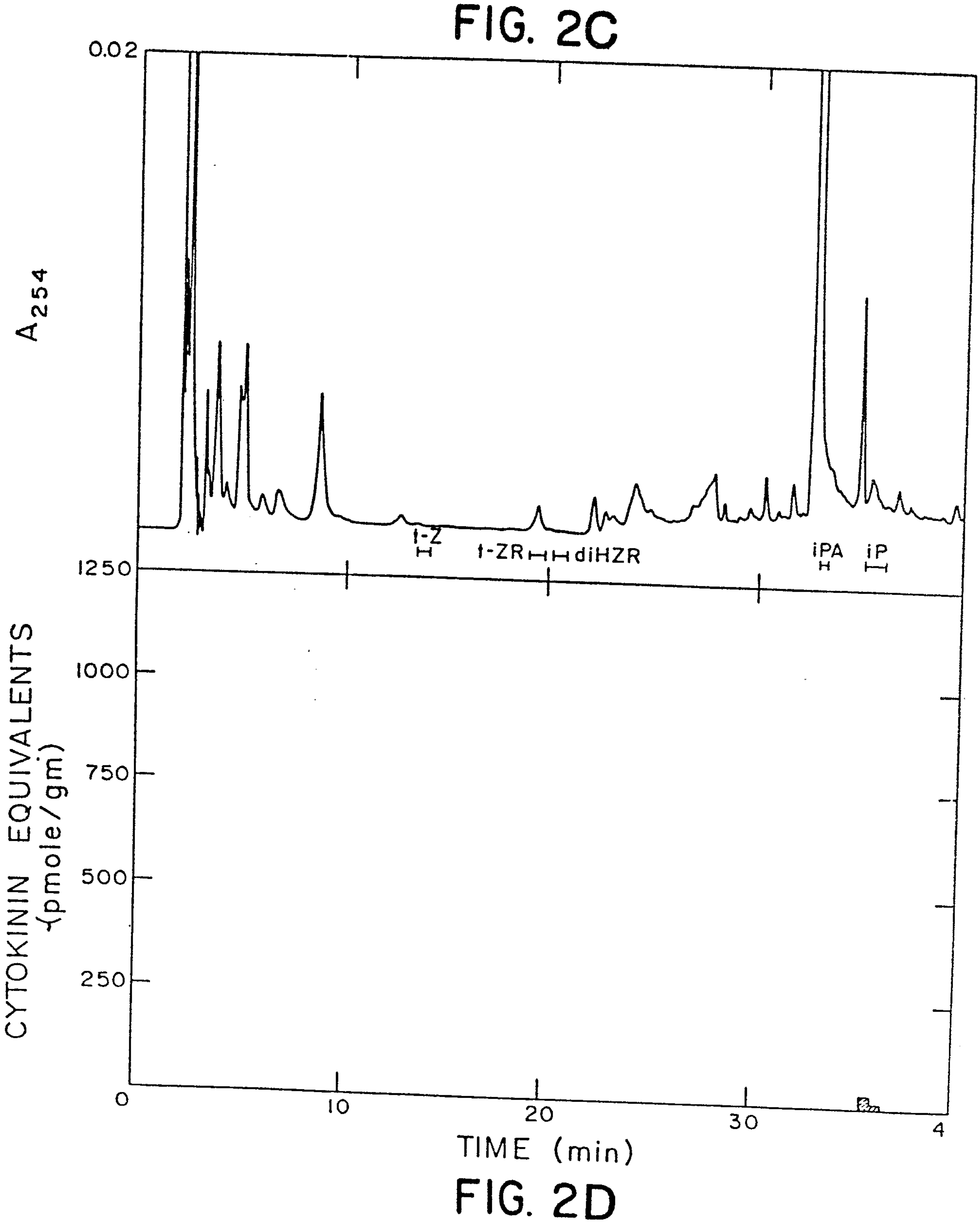
0.02
A254
t-Z
t-ZR
diHZR
iPA
iP
1250
1000
750
500
250
0
CYTOKININ EQUIVALENTS (pmole/gm)
10
20
30
4
TIME (min)

FIG. 2D

FIG. 3

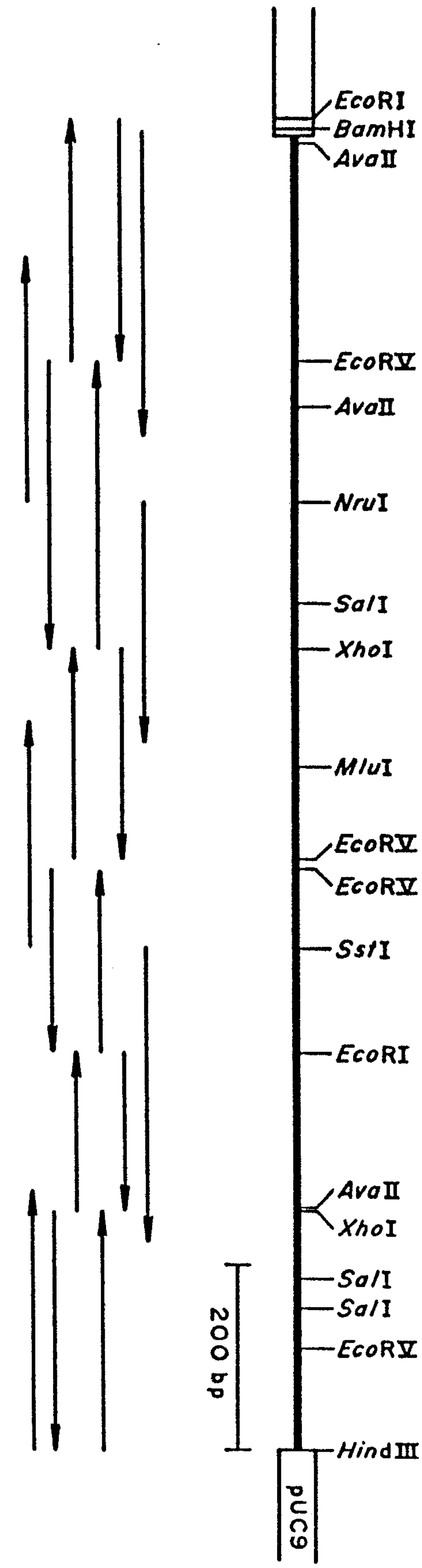
EcoRI
BamHI
AvaII
EcoRV
AvaII
NruI
SalI
XhoI
MluI
EcoRV
EcoRV
SstI
EcoRI
AvaII
XhoI
SalI
SalI
EcoRV
HindIII
200 bp
pUC9

FIG. 4-1

```
AACGGGGTCCGCTGCATTCACCATAAGCAGGTGTCGGGGCCAAACTCCTCAATTGTTCAGGAGGACAACGGGATGGAAA   -178

CTCTCATGGCCGAATGAGCCGGCAACGTCAAAAATGTACGATTACAAATGTAGCGCGATATTAGTTCAGCAGATTGCGT   -99

AAAGCTGGTAAAAATCATGCAGTAAAACATCATAATCCGAATTGAAAAATCTGGTTTGGAACAGATGGGATATCCCATT   -20

                     1
AAATTTATATGAGGCGCAC ATG TTA CTC CAT CTC ATC TAC GGA CCG ACT TGC AGC GGC AAA ACG   45
           SD        Met Leu Leu His Leu Ile Tyr Gly Pro Thr Cys Ser Gly Lys Thr

GAC ATG GCG ATC CAA ATC GCA CAA GAA ACC GGG TGG CCG GTG GTT GCC CTT GAT CGT GTG   105
Asp Met Ala Ile Gln Ile Ala Gln Glu Thr Gly Trp Pro Val Val Ala Leu Asp Arg Val

CAA TGC TGT CCT CAA ATC GCG ACA GGT AGC GGA AGA CCT TTG GAA TCG GAA TTG CAA TCA   165
Gln Cys Cys Pro Gln Ile Ala Thr Gly Ser Gly Arg Pro Leu Glu Ser Glu Leu Gln Ser

ACG CGG AGA ATA TAT TTG GAT TCC CGC CCC CTC ACC GAG GGC ATC CTT GAC GCT GAG AGT   225
Thr Arg Arg Ile Tyr Leu Asp Ser Arg Pro Leu Thr Glu Gly Ile Leu Asp Ala Glu Ser

GCC CAT CGT CGA CTC ATA TTC GAA GTG GAT TGG CGG AAG TCC GAA GAG GGT CTT ATT CTC   285
Ala His Arg Arg Leu Ile Phe Glu Val Asp Trp Arg Lys Ser Glu Glu Gly Leu Ile Leu

GAG GGC GGG TCG ATT TCG CTT CTC AAT TGC ATG GCT AAA AGT CCG TTT TGG AGA TCG GGT   345
Glu Gly Gly Ser Ile Ser Leu Leu Asn Cys Met Ala Lys Ser Pro Phe Trp Arg Ser Gly

TTT CAA TGG CAT GTC AAG CGG CTA CGT CTT GGG GAT TCG GAC GCC TTT CTC ACC CGA GCC   405
Phe Gln Trp His Val Lys Arg Leu Arg Leu Gly Asp Ser Asp Ala Phe Leu Thr Arg Ala

AAG CAA CGC GTT GCG GAA ATG TTT GCC ATC CGG GAA GAT CGC CCC TCG TTG TTG GAG GAG   465
Lys Gln Arg Val Ala Glu Met Phe Ala Ile Arg Glu Asp Arg Pro Ser Leu Leu Glu Glu
```

6/16 248984

FIG. 4-2

```
TTG GCG GAA CTC TGG AAC TAC CCT GCC GCT CGA CCG ATT TTG GAA GAT ATC GAC GGA TAT    525
Leu Ala Glu Leu Trp Asn Tyr Pro Ala Ala Arg Pro Ile Leu Glu Asp Ile Asp Gly Tyr

CGC TGC GCA ATT CGT TTT GCG CGC AAA CAC GAT CTC GCA ATC AGC CAG TTG CCA AAT ATT    585
Arg Cys Ala Ile Arg Phe Ala Arg Lys His Asp Leu Ala Ile Ser Gln Leu Pro Asn Ile

GAT GCA GGG CGG CAC GTA GAG CTC ATA GAG GCC ATA GCT AAT GAA TAT CTT GAA CAT GCG    645
Asp Ala Gly Arg His Val Glu Leu Ile Glu Ala Ile Ala Asn Glu Tyr Leu Glu His Ala

CTC TCG CAG GAG CGC GAT TTT CCT CAG TGG CCA GAA GAT GGC GCA GGA CAG CCT GTT TGC    705
Leu Ser Gln Glu Arg Asp Phe Pro Gln Trp Pro Glu Asp Gly Ala Gly Gln Pro Val Cys

CCG GTC ACG CTG ACG CGA ATT CGG TGA TTCGCCGCTTCATCATTTGACCTGCGTGTTGGATGGCCATCGG    775
Pro Val Thr Leu Thr Arg Ile Arg END

CGGCGCAGCATCGGTCGGCGTCACCTCCAAGCGCGACATGCGGCGCGCCAAAGCTATTCGCACATCTTATTTGAGTAAG    854

TGATTTCTTTCGAAGCACCTCGGCCGGCGCTCGGTCCTCGAGGGATTTGGCCGCTTCCGAGTTCCAGCAGTTGCAGATT    933

TCGATTAGCCGGTGTCGGTCAGGCTTCAAAGTCGACTTCCATCAAAGTCATTTTGGTTACACGTCGACATTGCATCTCA    1012

GCAAGACGAGTGGCTGTGCCGACTGATATCGACCGATTGCCATTACGTACAGCAGGCTTATTCGGTTACATTTGAAACC    1091

AATTTTTGGTCTCCTGTAATCATTATACTGCTGATGGACTGCACATATCGTCGGTA
```

```
tmr      10        20        30        40        50        60        70        80        90        100       110
ATGTTACTCCATCTCATCTACGGACCGACTTGCAGCGGCAAAACGGACATGGCGATCCAAATCGCACAAGAAACCGGTGGCCGGTGGTTGCCCTTGATCGTGTGCAATGCTGTCCTCAA
:::  :: ::::: :: : ::: :: :::::::  :: :: :::   :  :::::      : :: ::  : :: :::   :: ::  :: : :::::::: :: ::::::::::::::
ATGGACCTGCATCTAATTTTCGGTCCAACTTGCACAGGAAAGACGACGACCGCGATAGCTCTTGCCCAGCAGACAGGGCTTCCAGTCCTTTCGCTTGATCGGGTCCAATGCTGTCCTCAA
tzs      10        20        30        40        50        60        70        80        90        100       110

         130       140       150       160       170       180       190       200       210       220       230
ATCGCGACAGGTAGCGGAAGACCTTTGGAATCGGAATTGCAATCAACGCGGAGAATATATTTGGATTCCCGCCCCCTCACCGAGGGCATCCTTGACGCTGAGAGTGCCCATCGTCGACTC
 :  : :: :: :::::: ::::        :   ::: :: ::  ::::  : :  : ::  : :::    :: :: ::    ::::: ::: : :   ::  ::   :: :::: : : ::
CTATCAACCGGAAGCGGACGACCAACAGTGGAAGAACTGAAAGGAACGACGCGTCTCTACCTTGATGATCGGCCTCTGGTGGAGGGTATCATCGCAGCCAAGCAAGCTCATCATAGGCTG
         130       140       150       160       170       180       190       200       210       220       230

         250       260       270       280       290       300       310       320       330       340       350
ATATTCGAAGTGGATTGGCGGAAGTCCGAAGAGGGTCTTATTCTCGAGGGCGGGTCGATTTCGCTTCTCAATTGCATGGCTAAAAGTCCGTTTTGGAGATCGGGTTTTCAATGGCATGTC
::    :: ::: ::   :    :: :: : :  :: :::::::: ::::: :: :: :   ::: : ::::: ::::::::    ::         : ::::::  : : :::::  :::::: :
ATCGAGGAGGTGTATAATCATGAGGCCAACGGCGGGCTTATTCTTGAGGGAGGATCCACCTCGTTGCTCAACTGCATGGCGCGAAACAGCTATTGGAGTGCAGATTTTCGTTGGCATATT
         250       260       270       280       290       300       310       320       330       340       350

         370       380       390       400       410       420       430       440       450       460       470
AAGCGGCTACGTCTTGGGGATTCGGACGCCTTTCTCACCCGAGCCAAGCAACGCGTTGCGGAAATGTTTGCCATCCGGGAAGATCGCCCCTCGTTGTTGGAGGAGTTGGCGGAACTCTGG
: .:: :      :     ::    :: :::: : :      ::::::   : :::  : : :::::   :   :   : ::   :::  ::  :   :   : :::::::    : :: :::
ATTCGCCACAAGTTACCCGACCAAGAGACCTTCATGAAAGCGGCCAAGGCCAGAGTTAAGCAGATGTTGCACCCCGCTGCAG---GCCATTCTATTATTCAAGAGTTGGTTTATCTTTGG
         370       380       390       400       410       420       430       440          450       460       470

         490       500       510       520       530       540       550       560       570       580       590
AACTACCCTGCCGCTCGACCGATTTTGGAAGATATCGACGGATATCGCTGCGCAATTCGTTTTGCGCGCAAACACGATCTCGCAATCAGCCAGTTGCCAAATATTGATGCAGGGCGGCAC
::  : :::          : :: ::: :: :::: ::::: :::::::: :   :: ::    :::::  :: :  :: :  :: :          : :     :  :::: :::      : :
AATGAACCTCGGCTGAGGCCCATTCTGAAAGAGATCGATGGATATCGATATGCCATGTTGTTTGCTAGCCAGAACCAGATCACGGCAGATATGCTATTGCAGCTTGACGCAAATATGGAA
  480      490       500       510       520       530       540       550       560       570       580       590

         610       620       630       640       650       660       670       680       690       700       710
GTAGAGCTCATAGAGGCCATAGCTAATGAATATCTTGAACATGCGCTCTCGCAGGAGCGCGATTTTCCTCAGTGGCCAGAAGATGGCGCAGGACAGCCTGTTTGCCCGGTCACGCTGACG
:  :: : ::  : :  :: ::: : :: ::: :   ::::::: :   ::::: :   : :: :: ::        :                 ::::: :::   :::   ::  :
GGTAAGTTGATTAATGGGATCGCTCAGGAGTATTTCATCCATGCGCGCCAACAGGAACAGAAATTCCCCCAAGTTAACG--------------CAGCCGCTTTCGACGGATTCGAAGGTC
  600      610       620       630       640       650       660       670                     680       690       700

     X
CGAATTCGGTGA
   :  :  :
ATCCGTTCGGAATGTATTAG
      710       720X
```

FIG. 5

```
tmr   MDLHLIFGPTCTGKTTTAIALAQQTGLPVLSLDRVQCCPQLSTGSGRPTVEELKGTTRLY
         ::::.::::.:::. ::..::.:: ::..::::::::::..::::::  .:::..: :.:
tzs   MLLHLIYGPTCSGKTDMAIQIAQETGWPVVALDRVQCCPQIATGSGRPLESELQSTRRIY

tmr   LDDRPLVEGIIAAKQAHHRLIEEVYNHEANGGLILEGGSTSLLNCMARNSYWSADFRWHI
      ::.:::.:::..:. ::.::: ::    ....::::::::.::::::::...:...:.::.
tzs   LDSRPLTEGILDAESAHRRLIFEVDWRKSEEGLILEGGSISLLNCMAKSPFWRSGFQWHV

tmr   IRHKLPDQETFMKAAKARVLQMLHPAAGH-SIIQELVYLWNEPRLRPILKEIDGYRYAML
        : .: : ..:.. ::.:: .:.   ... :...::. ::: :  ::::.:::::.:.
tzs   KRLRLGDSDAFLTRAKQRVAEMFAIREDRPSLLEELAELWNYPAARPILEDIDGYRCAIR

tmr   FASQNQITADMLLQLDANMEGKLINGIAQEYFIHARQQEQKFPQVNAAAFDGFEGHPFGMY
      ::...... . : ..::... .::..::.::. ::  ::..:::
tzs   FARKHDLAISQLPNIDAGRHVELIEAIANEYLEHALSQERDFPQWPEDGAGQPVCPVTLTRIR
```

FIG. 6

11.07.87 0248984

10/16

FIG. 7

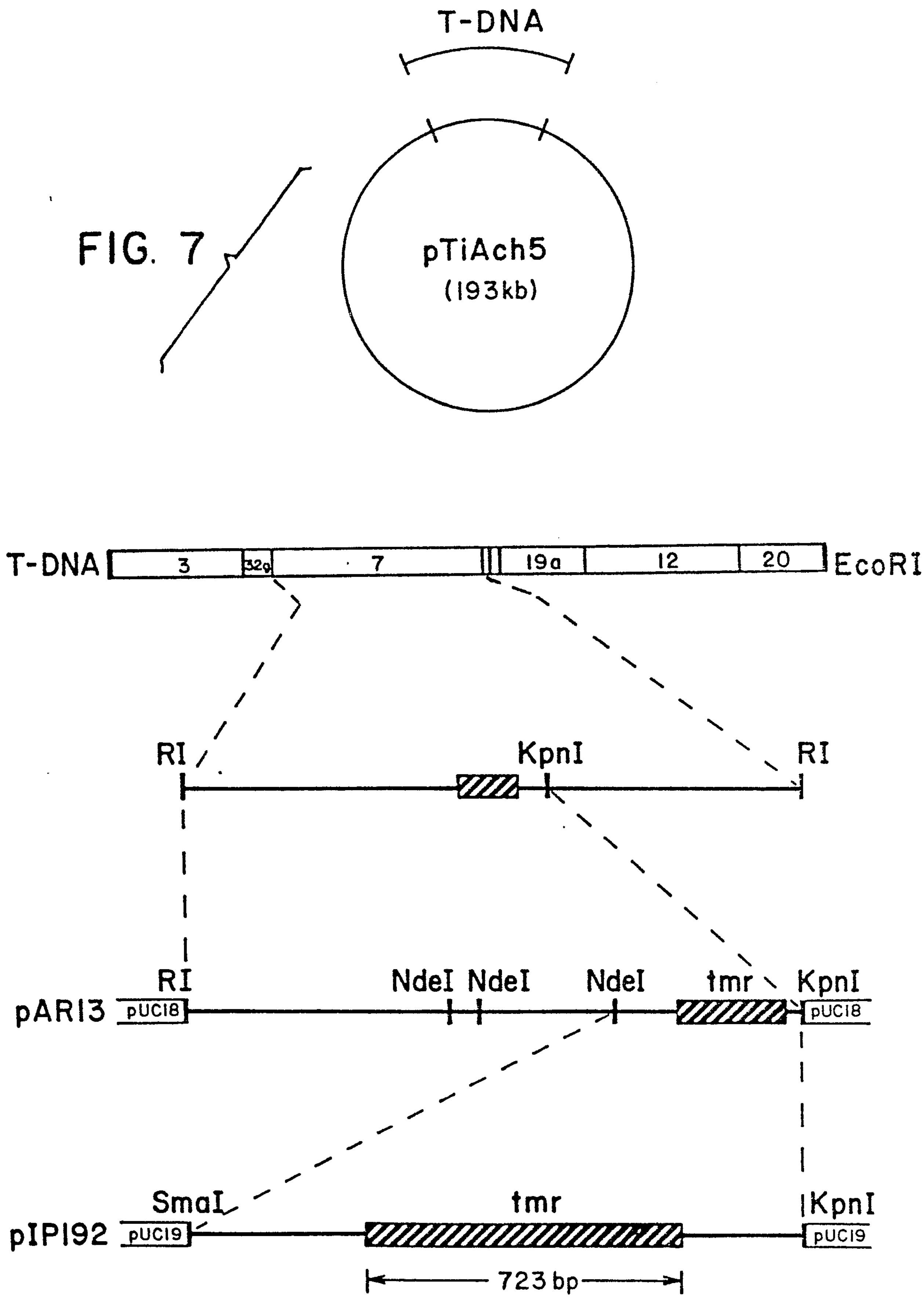

11.07.87

0248984

11/16

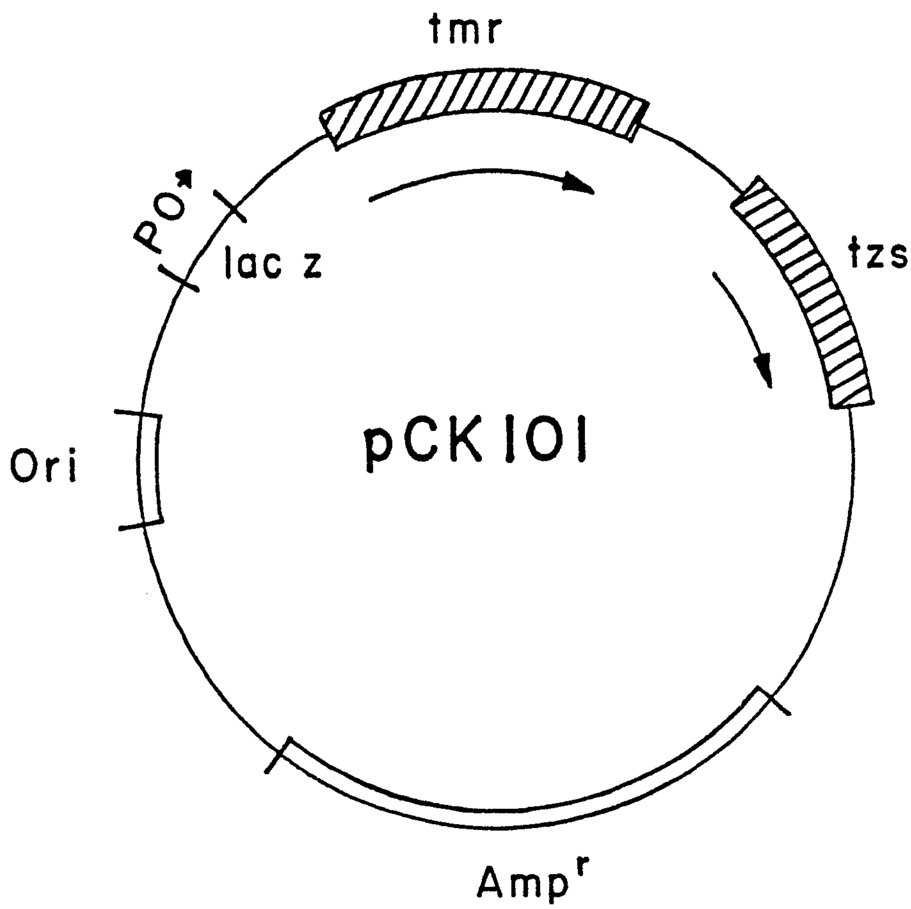


FIG. 8

FIG. 9A

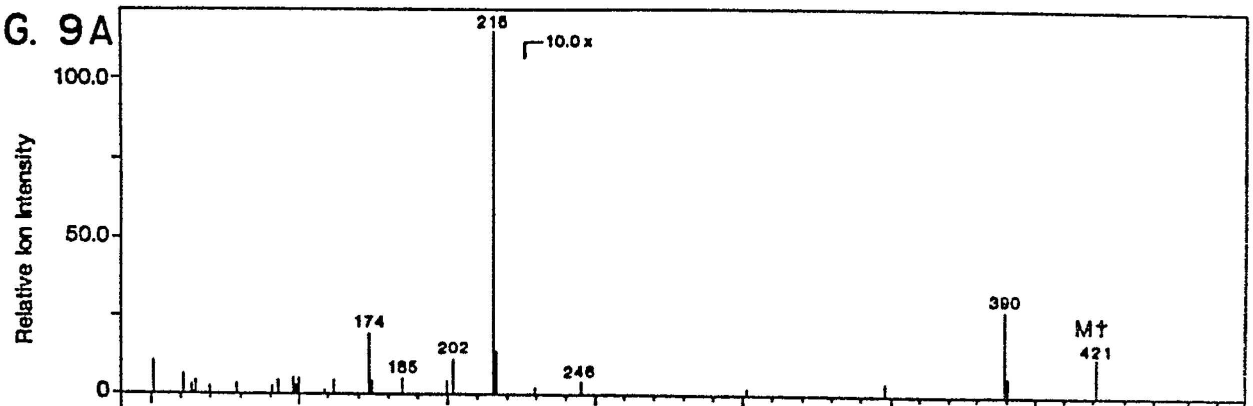


FIG. 9B

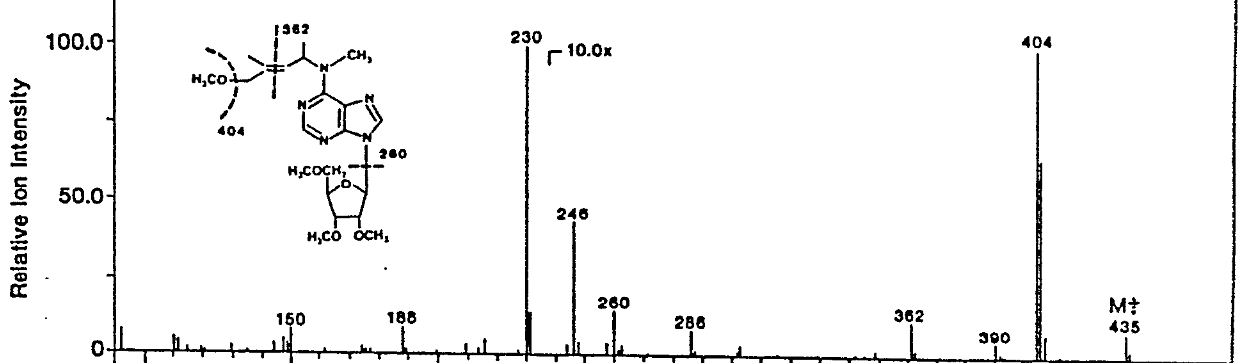


FIG. 9C

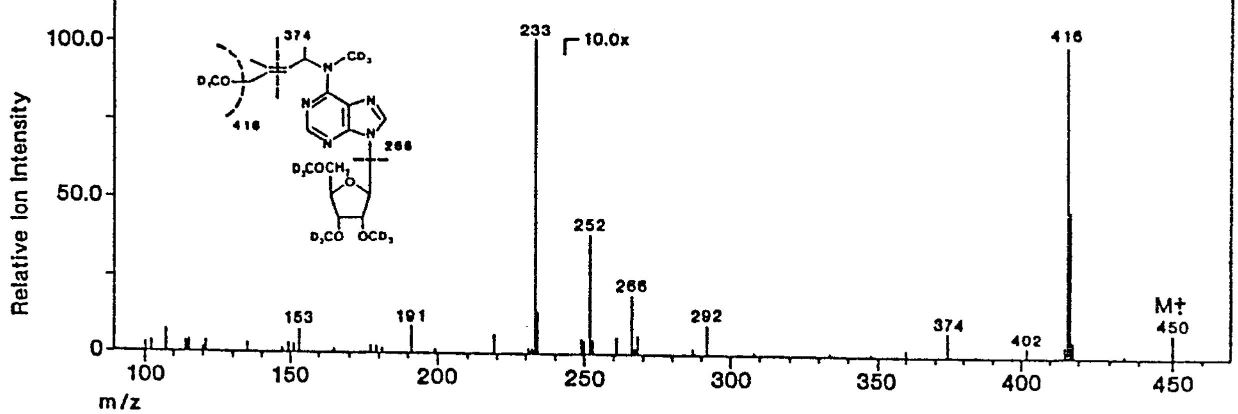

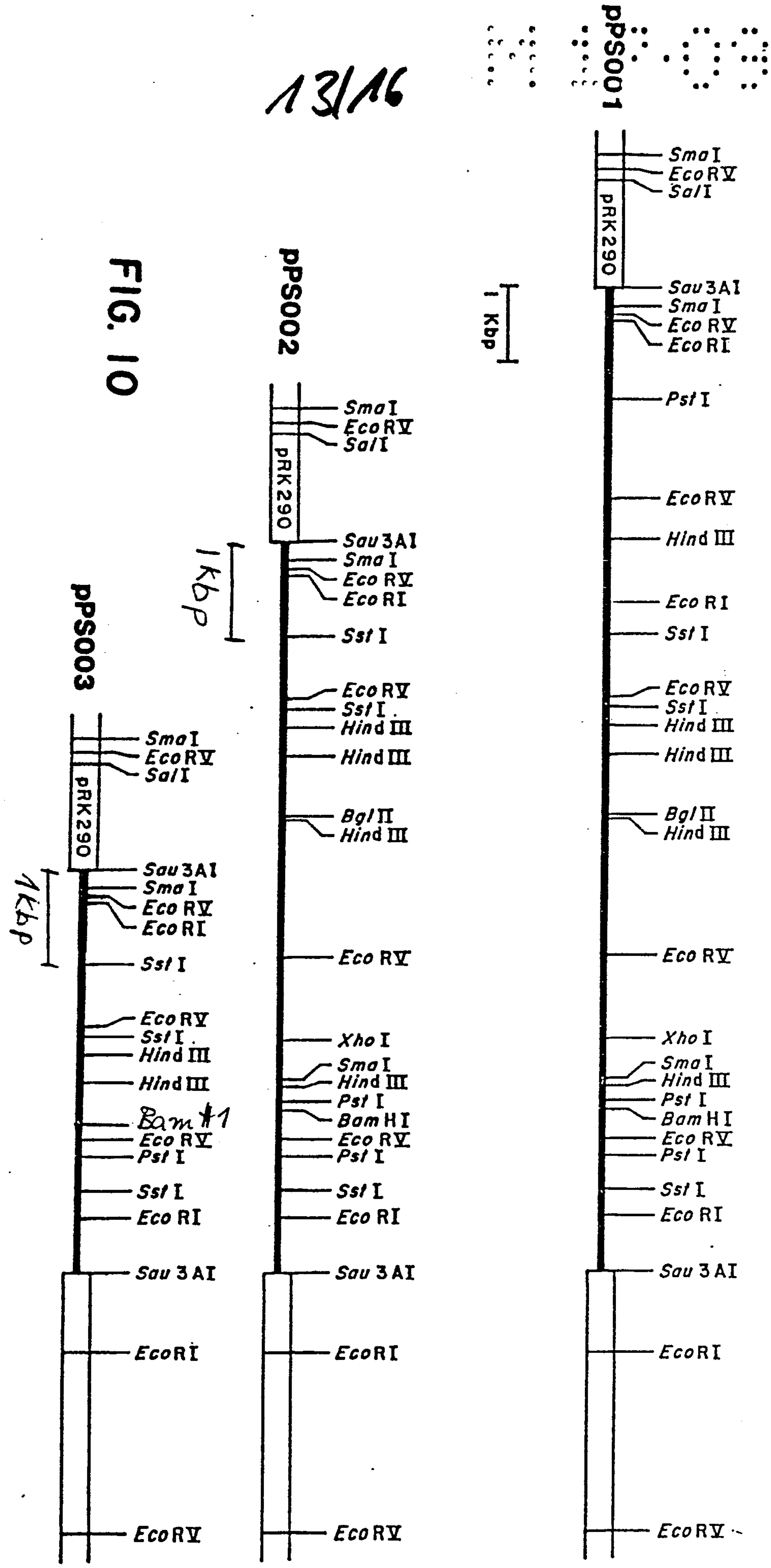
pPS001
pRK290
Sma I
Eco RV
Sal I
Sau 3AI
Sma I
Eco RV
Eco RI
Pst I
Eco RV
Hind III
Eco RI
Sst I
Eco RV
Sst I
Hind III
Hind III
Bgl II
Hind III
Eco RV
Xho I
Sma I
Hind III
Pst I
Bam HI
Eco RV
Pst I
Sst I
Eco RI
Sau 3AI
Eco RI
Eco RV
1 Kbp
pPS002
pRK290
Sma I
Eco RV
Sal I
Sau 3AI
Sma I
Eco RV
Eco RI
1 Kbp
Sst I
Eco RV
Sst I
Hind III
Hind III
Bgl II
Hind III
Eco RV
Xho I
Sma I
Hind III
Pst I
Bam HI
Eco RV
Pst I
Sst I
Eco RI
Sau 3AI
Eco RI
Eco RV
pPS003
pRK290
Sma I
Eco RV
Sal I
Sau 3AI
Sma I
Eco RV
Eco RI
1 Kbp
Sst I
Eco RV
Sst I
Hind III
Hind III
Bam #1
Eco RV
Pst I
Sst I
Eco RI
Sau 3AI
Eco RI
Eco RV

FIG. 10

FIG. 11A

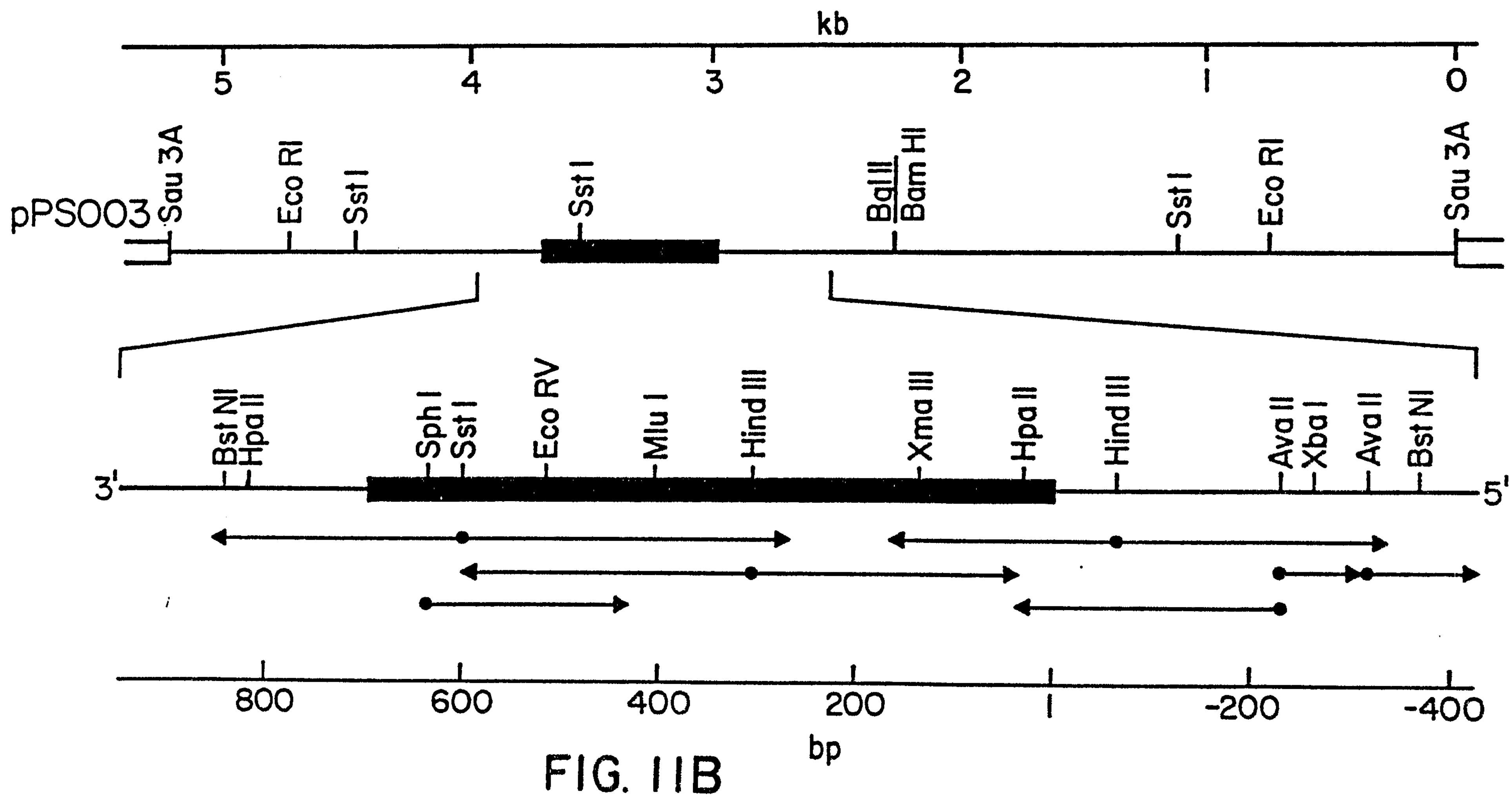


FIG. 11B

FIG. 12-1

```
CTATGCAGTGCAAACGAAGCGCAAGTTGAATGGAACGGCTGATCTTCAGTTCTCATTTAAATAATATGCTGAGCCTGGC -554
AGAGTTAGAATCAACTTCTGCTTCAATCTTATCGCCTCTGATTACAAGGCCTCATAGGTATATAATTCCAGGAACGAAT -475
AGAGGGTGATTTTGTATAAAGACGAATGTATTAGGCAGGCAGCCTTAATATCAAGCAGGAGCGATATAATCTGCTTGTA -396
TCTGTCTCCACATGCCAGCCGTGACCTGGTTTTTAGAGCAGCGCAAGACACTTTAATCGCTATGCCGATCTTGAGTAAT -317
CGGACCTGTCTGCTTTATACTATTAGTAATTGTTTTTCGGATTGATCACATTCTAGATCAGTGCAAACGCAAGCGCAAG -238
TTGAATGGAACGGTCCAGAAGAAAAGTCGAACTGTAAAAGTGGAATGCTCTTTTCTTCTGGTTGGAGGGAAGGAGTTTC -159
CGAGCATGTTTCTTTACGGTTGGCACGGTTTGCGAGTGAATGTTGAATTTAGTTTGGCTAGAGTTTTAGATTGGGTATA  -80
ATGTTGGGAGGGGGCAGCCAAGCTTGTGTTTCAAAAAACGGATAGTTTTTTTGCAAACTCGCCTGATTAGGAGGTGCGG  -1
ATG AAA ATA TAT TTA ATA TGG GGT GCT ACT TGT ACC GGA AAG ACC GAG CAT TCT ATT AAG  60
Met Lys Ile Tyr Leu Ile Trp Gly Ala Thr Cys Thr Gly Lys Thr Glu His Ser Ile Lys  20

TTA TCA AAG AGT ACT GGT TGG CCA GTC ATA GTC TTG GAT AGG GTT CAG TGC TGT TTC GAT  120
Leu Ser Lys Ser Thr Gly Trp Pro Val Ile Val Leu Asp Arg Val Gln Cys Cys Phe Asp  40

ATT GCC ACA GGT AGT GGG CGG CCG CAT CCT GAA GAG CTA CAA TCA ACG CGG CGT ATA TAC  180
Ile Ala Thr Gly Ser Gly Arg Pro His Pro Glu Glu Leu Gln Ser Thr Arg Arg Ile Tyr  60

TTA GAT AAT CGT CGC ATT TCT GAA GGG GTT ATA AGT GCA GAA GAA GCT AAT GAC AGG CTC  240
Leu Asp Asn Arg Arg Ile Ser Glu Gly Val Ile Ser Ala Glu Glu Ala Asn Asp Arg Leu  80
```

FIG. 12-2

AAG TTG GAA GTA AAT AAA CAT ATA GAT AGT GGT GGG GTT ATT CTT GAG GGG GGG TCG ATT 300
Lys Leu Glu Val Asn Lys His Ile Asp Ser Gly Gly Val Ile Leu Glu Gly Gly Ser Ile 100

TCG TTG TTG AAG CTT ATT TCT AAA GAC CCA TAC TGG TGC GAT AGA TTT ATA TGG AGC CAG 360
Ser Leu Leu Lys Leu Ile Ser Lys Asp Pro Tyr Trp Cys Asp Arg Phe Ile Trp Ser Gln 120

CAT AGA ATG AGA TTG CAA GAT ACC GAT GTA TTC ATG GAT AAA GCC AAA GCA CGC GTT AGG 420
His Arg Met Arg Leu Gln Asp Thr Asp Val Phe Met Asp Lys Ala Lys Ala Arg Val Arg 140

CGT ATG CTT GTA GGC TCT ACA GAA ACC ACG GGG TTG TTA GAC GAG TTG GTT GCG GCT CAG 480
Arg Met Leu Val Gly Ser Thr Glu Thr Thr Gly Leu Leu Asp Glu Leu Val Ala Ala Gln 160

TCA GAC CTT AAT GCT AAA CTC GCT ATA CAA GAT ATT GAC GGA TAT CGA TAT ATT ATG AAC 540
Ser Asp Leu Asn Ala Lys Leu Ala Ile Gln Asp Ile Asp Gly Tyr Arg Tyr Ile Met Asn 180

TAT GCT CAG GCG CGT CGC CTC AGT ATC ACT CAA CTT CTG AAT GTG ATG ACT GGT GAT ATG 600
Tyr Ala Gln Ala Arg Arg Leu Ser Ile Thr Gln Leu Leu Asn Val Met Thr Gly Asp Met 200

AAA GAG GAG CTC ATT AAT GGA ATA GCA TTA GAA TAC TAT GAG CAT GCT AAA TGG CAA GAG 660
Lys Glu Glu Leu Ile Asn Gly Ile Ala Leu Gly Tyr Tyr Glu His Ala Lys Trp Gln Glu 220

CGC GAT TTC CCT GCG GAG TGG CTC GCT GAA CGT AGT ACA CGG TAA GGAGGATAAGACTGCAAAG 724
Arg Asp Phe Pro Ala Glu Trp Leu Ala Glu Arg Ser Thr Arg *** 234

GTTCAGCATAGGAATTTCAAAATGCCTATGCATGAAGCTGACGCTGTGAAGGAGGCGTCATGCTTTCATCGGCATAGGG 803

TGCAGCCATCTGGGCAGCCCTGCCACACACCGGCGCACGATGTCGGCCTGGCTGGGAGCCTAGA 867